# EUROPEAN PATENT SPECIFICATION

(11) **EP 3 064 596 B1**
(45) Date of publication and mention of the grant of the patent: **30.01.2019**
(21) Application number: 16157930.5
(22) Date of filing: 29.02.2016
(51) Int. Cl.: C12Q 1/68

(54) **METHOD FOR ANALYSING CYP2C19 GENE POLYMORPHISM, KIT AND USE THEREOF FOR ANALYSING THE CYP2C19 GENE POLYMORPHISMS AND TO EVALUATE DRUG EFFICACY.**
VERFAHREN ZUR ANALYSE VON CYP2C19-GENPOLYMORPHISMUS, KIT UND DESSEN VERWENDUNG ZUR ANALYSE VON CYP2C19-GENPOLYMORPHISMUS UND ZUR EVALUIERUNG DER ARZNEIMITTELWIRKSAMKEIT
PROCÉDÉ D'ANALYSE DES POLYMORPHISMES DU GENE CYP2C19, KIT ET SON UTILISATION POUR ANALYSSER LES POLYMORPHISMES DU GENE CYP2C19 ET EVALUER L'EFFICACITE DE DROGUES.

(30) Priority: 05.03.2015 JP 2015043933
(43) Date of publication of application: 07.09.2016
(73) Proprietor: ARKRAY, Inc., Minami-ku Kyoto-shi, Kyoto 601-8045 (JP)
(72) Inventor: TAZOE, Hideaki, Kyoto-shi, Kyoto 602-0008 (JP)
(74) Representative: Dehns

(56) References cited:
- EP-A1- 2 615 182
- CN-A- 102 776 279
- CN-A- 103 468 818
- Anonymous: "Job result: sequence 3", , 15 June 2016 (2016-06-15), XP055280524, Retrieved from the Internet: URL:http://ibis.internal.epo.org/exam/jobR esult?id=394367 [retrieved on 2016-06-15]
- Anonymous: "Job result: sequence 4", , 15 June 2016 (2016-06-15), XP055280526, Retrieved from the Internet: URL:http://ibis.internal.epo.org/exam/jobR esult?id=394368 [retrieved on 2016-06-15]
- Anonymous: "Job result: sequence 5", , 15 June 2016 (2016-06-15), XP055280527, Retrieved from the Internet: URL:http://ibis.internal.epo.org/exam/jobR esult?id=394369 [retrieved on 2016-06-15]
- Anonymous: "Job result: sequence 6", , 15 June 2016 (2016-06-15), XP055280529, Retrieved from the Internet: URL:http://ibis.internal.epo.org/exam/jobR esult?id=394370 [retrieved on 2016-06-15]
- Hye-Eun Jeong ET AL: "Development of a Multiplex and Cost-Effective Genotype Test toward More Personalized Medicine for the Antiplatelet Drug Clopidogrel", International Journal of Molecular Sciences, vol. 15, no. 5, 5 May 2014 (2014-05-05), pages 7699-7710, XP055417111, DOI: 10.3390/ijms15057699

### SEQUENCE LISTING

<110> ARKRAY, INC.
<120> Primer Set for Amplifying Cyp2c19 Gene, Kit for Amplifying Cyp2c19 Gene, Gene Amplification Method, Polymorphism Analysis Method, and Drug Efficacy Evaluation Method
<130> 42.127727
<141> 29 February 2016
<150> JP2015-043933
   <151> 05 March 2015
<160> 69
<210> 1
   <211> 1474
   <212> DNA
   <213> Homo sapiens
<400> 1
   cttcaccctg tgatcccact ttcatcctgg gctgtgctcc ctgcaatgtg atctgctcca      60
   ttattttcca gaaacgtttc gattataaag atcagcaatt tcttaacttg atggaaaaat     120
   tgaatgaaaa catcaggatt gtaagcaccc cctggatcca ggtaaggcca agttttttgc      180
   ttcctgagaa accacttaca gtcttttttt ctgggaaatc caaaattcta tattgaccaa     240
   gccctgaagt acatttttga atactacagt cttgcctaga cagccatggg gtgaatatct     300
   ggaaaagatg gcaaagttct ttattttatg cacaggaaat gaatatccca atatagatca     360
   ggcttctaag cccattagct ccctgatcag tgttttttcc actaaactcc aaagccctgt      420
   ttctataaag tactttggtg acagccccaa agcgtgctta tatcactcca tggacatcca     480
   ggcactttgg agtcttccat tactcacaag gcttgtcctt caattcacac tttgtcatat     540
   tgtgtgacag aaatatccta atctaaaaga cattatctcc ttcaaggaca gagaatattt     600
   ggaaccacag aagctgccaa gaaacactga atagggcaga ggtgtttgat gtctcagttg     660
   ggattctagc tgatgagaca gctggttagg aatgaaaaaa ttattgtttt tttggtgtat     720
   gaaccataaa cagacatcac acttttaccc tgtgctgaat tggcatgttt tatactctgc     780
   ctaaataata attgtatgat tttacagaag tcatttaact gctctggtgc acagttggaa     840
   tttgaagtat ctttgagccc ctcccacttc taaaatacta attcaatttc agaggctgct     900
   tgatagaaat caatatagca gggactatct ttgtagtatc aatcaggttg tgcaaactct     960
   tttaacctat gctatcatct ccaaaatgtt aatgtagtaa ttcataccat cttatatttc    1020
   aagattgtag agaagaattg ttgtaaaaag taagagaatt aatataaaga tgcttttata    1080
   ctatcaaaag caggtataag tctaggaaat gattatcatc tttgattctc ttgtcagaat    1140
   tttctttctc aaatcttgta taatcagaga attactacac atgtacaata aaaatttccc    1200
   catcaagata tacaatatat tttatttata tttatagttt taaattacaa ccagagcttg    1260
   gcatattgta tctatacctt tattaaatgc ttttaattta ataaattatt gttttctctt    1320
   agatatgcaa taattttccc actatcattg attatttccc gggaacccat aacaaattac    1380
   ttaaaaacct tgcttttatg gaaagtgata ttttggagaa agtaaaagaa caccaagaat    1440
   cgatggacat caacaaccct cgggacttta ttga                                1474
<210> 2
   <211> 5000
   <212> DNA
   <213> Homo sapiens
<400> 2
   ggcctgtttt cctgggtatc accagcagag gctgcagaac agcaaatatt gcagaacagc 60
   aagtattgct gcttgatcct tcctctggaa gcttcatctc ggaggggcac ctggctgtgt 120
   gaggtgtcag tcagccccta ctaggaggtg tctcccagtt aggctacatg ggggtctggg 180
   acccacttga ggagacagtc tgtctgttct cagagctcaa acactgtgct gggagagcca 240
   ctgctgtctt caaagctgtc agacagggat gtttaagtct gcagaagttt ctgctgccat 300
   ttgtttagca atgccctgcc cccagaggtg gagtctacag aggcaggcag gcctggttga 360
   gctgtggtgg gctctaccca gcttgagctt cctggctgct ttgtttatgt actcaagcct 420
   cagcaatggt ggatgcccct cccccaacca gactgccacc ttgcagttcg aactcagatt 480
   gctgtcctag cagtgaacaa ggctccgtgg gcatgggacc tgctgagcca ggcacagtgt 540
   agaatctcct aatgtgccat ttgctaagac cattggggaa gcacagtatt tgggtggcag 600
   tgtcctgatt ttcccagtac agtctctcat ggcttccctt gtctagaaaa gggaaatccc 660
   ccaacctctt gtgctttctg ggtgaggcga tgccccaccc tgttttggct tgccctacat 720
   gggctgcacc cactgtccaa ctggtcccaa tgagatgaac aagatacctc agttggaatt 780
   gcaaaaatca gccatcttct gcatcaatca ccctgggagc tgcagaccgg agctgttcgt 840
   atttggccat cttggaatga ggggcctcta tttttattat tttttatctt tagatattaa 900
   cagtctgatt gtaaagttta tatggagagg ccaaaaaacc cagaacagcc aatgttaagg 960
   agaacaaaat caagtgatta accttgtctg attcaacttc aagacttatt atgaagctgc 1020
   agtaatcaag acaatgtgaa attggtgaag aaacagtcaa atagattaat gcaagtgagt 1080
   aaagagcctg aaatagataa tcagaaatac agccaactga tctttgaaaa aaaagcaaag 1140
   gcaattcaat ggggaataat agtcttttcc acaaagacta atggagtaac tggagattca 1200
   catacagaaa aatgaatctg gaaatagacc ttacatcttt aacgcaagtt aactaaaaat 1260
   atattataga tctaaatact aaatgcaaaa ctatgaaact ctggtaatat aaggtaggag 1320
   aaaataaaga tgaccttgat ctggcaatgg tttttaaaat actacaatga aggcacaatc 1380
   catgaaataa agaataacta agctggcctt tattaaaatt taaaacttct actccacaaa   1440
   agatagtatc aagggaataa gatgacaaga cacagactgg gataaatatt tttcaaaaga   1500
   tatatctgat aaatgatggc tatccaaaat ttttaaaatt caacaattag aaatcagaca   1560
   ccttgattaa aaaaatgggc aacgggtctg aacagacacc tcaccaagaa gacatacaga   1620
   taccaaataa atatatgaaa agtactaatc attaaatgtc attagggaac tgcaagctaa   1680
   aaccccgatg acatactatt acacacttat acactggcta aaattcaaaa cactgataga   1740
   accaaacact ggcaaggata tgcagcaagg agaactatta gtcattgctg gtggaaatgc   1800
   aaaatggtac agacacttca ggagacagtt gggcagtatc taataaaact aaaatatgtt   1860
   tactatatta tctagtagtt gtgctccttg gcatttgcca aatgaattga aaatatattt   1920
   ccacacaaaa atctgcacat ggatgtttat agcagtttta ttcatcattg tcaaaaatta   1980
   gaaatcacca agatgttctt caatagataa atgaatagac aacatggtaa acctatacaa   2040
   tgaatgttat ttagtgttaa aagaaatgaa ctatcaagcc atgaaaagac atggagaaat   2100
   tttatacata ttagtaagtt aaatcttaaa aggctacata ctgtatgatt ccaaacatat   2160
   gacattctgg aaaaggcaaa actatgccaa cactgcaaag attatttgtt gctagggctc   2220
   gtgaggaaga gagggtgaac aggtagagca cagagggttt taagggcagt aaagctattt   2280
   tatatgatac tacaatggtg gatacatgtc attatgcatt agtcaaatcc catagaatgt   2340
   acaacacaaa gaatgaaccc cacagaaact atgagctttg gttgttgatg tgtcaatgtt   2400
   ggttcattga ttgtaaccta tgagagttct tgattgtgag aggctgtgtg tgggtgcagg   2460
   gaaagaggca tgtggtaatt atatactttc cactttccac ttagtgttgc tggaaacccc   2520
   aaactgctgt taagaaatac atttttctaa agcctacttt aacccaccat tctagtcaag   2580
   ctgaggtttg tattacccag tgactgtgga gggcttaatg ttgatactcc cctgatcact   2640
   tggactgaga tgtgaattct atgagacggg atgtgatatg tcactgaact gggagttgaa   2700
   aaactgtgat tctaagtctt atactgaggc attgtgatac tttgtctcac tgagtcaaca   2760
   attgctcatt taaaaaaaaa atcaattcct gtttagaata gttgatctca gagatccctt   2820
   ttgtcttcaa attatctata attctttcat agtataaact gaaagatcta gggaggagcc   2880
   tcaacttagc agaagagagg aggagcagaa ctggaacacc tagctctcaa ggaatgaaat   2940
   gattattcta aagagagcaa ccaagcttat cttacccaaa ctaaggtagt atatttctgt   3000
   tagagtttaa agttacgtga gtcagggacc aagttattgc ttttctttgc cctgtataaa   3060
   gggttctcca aggccttgga ctcacccaag tactaaaggt tataaaacca aactcttctg   3120
   acccccaatc tagtcaactt gggctgtaat tattaatgaa attaatattt attttgaaaa   3180
   tcatttacta gactgaatca cgaaatactg aaccattgta cacaatcagt aaatatctgt   3240
   ggactcaatt gaactgaatg ttttgcttga aatgaaacct tcaagatgca gggcttatga   3300
   attctagtct cagctctagc accagcagac accatgttct tggctacagt actgaatctt   3360
   caaggctcag cctcctcatt cctgagatgg gtcaatttta ttgtaagcaa aggcaattga   3420
   gagattccaa agggatatga ggtgtgagaa ttctctctaa atggggttag aatccctgtt   3480
   aaaaatgacc agtgaaacat tgtgcaattg tgtcttaaca taacttactt tttcttaata   3540
   agagaactgg aaataacctc attaggaaat ttagaacaaa tacgatgata tctttaaaga   3600
   aaatggcttt gtgtaagtat tgtcgttagt gatctagtaa tgtgtatctt tctggttgta   3660
   tttagacctt caactcaaat gtcagctccc gttaaggtct atacattgtg gtggttttgt   3720
   gctgtgggtc catttagtga tttccctacc tcccatcctc tattagattc acaactgttg   3780
   ttctgcccat aatttcctat gcttgctttg cattgttaca tttttttttg aaaatcagaa   3840
   agcaaaatca atataaagca gccatgtctg gaggagacca ggaggtcaag aagccttagt   3900
   ttctcaagcc cttagcacca aattctctga gatcagctct tccttcagtt acactgagcg   3960
   tttcccctct gcagtgatgg agaagggaga actcttattt tttctcatga gcatctctgg   4020
   ggctgttttc cttagataaa taagtggttc tatttaatgt gaagcctgtt ttatgaacag   4080
   gatgaatgtg gtatatattc agaataacta atgtttggaa gttgttttgt tttgctaaaa   4140
   caaagtttta gcaaacgatt ttttttttca aatttgtgtc ttctgttctc aaagcatctc   4200
   tgatgtaaga gataatgcgc cacgatgggc atcagaagac ctcagctcaa atcccagttc   4260
   tgccagctat gagctgtgtg gcaccaacag gtgtcctgtt ctcccagggt ctcccttttc   4320
   ccatttgaaa tataaaaaat aacaattcct gccttcacgt gtttttttag ggggttaaat   4380
   ggtaaaggtg tttatatctg ctaaggtaat ttacttgata tatgtttggt tattgaagat   4440
   atatgagtta tgttagctat ttcatgttta ggctgctgta tttttagtag gctatattaa   4500
   atagaggatt tcattataaa ggacaaagtc tcctaatctt cgatatagga ttgacatact   4560
   ttttaaatat acaaggcata gaatatggcc atttccgtta aatcataaat tcccaactgg   4620
   ttattaatct aagaattcag aattttaagt aattgttttt gcatcagatt gtttacttca   4680
   gtgctctcaa ttatgacggt gcattggaac cacttgggtt aacatttttt tgtttttatt   4740
   accaatacct aggcttcaac ctagtacaat gaaaccagaa tgtacagagt gggcactggg   4800
   acgaaggaga acaagaccaa aggacatttt atttttatct ctatcagtgg gtcaaagtcc   4860
   tttcagaagg agcatatagt gggcctaggt gattggccac tttatccatc aaagaggcac   4920
   acacacttaa ttagcatgga gtgttataaa aagcttggag tgcaagctca cggttgtctt   4980
   aacaagagga gaaggcttca                                               5000
<210> 3
   <211> 40
   <212> DNA
   <213> Artificial Sequence
<220>
   <223> CYP2C19*2-F
<400> 3
   taaattattg ttttctctta gatatgcaat aattttccca 40
<210> 4
   <211> 23
   <212> DNA
   <213> Artificial Sequence
<220>
   <223> CYP2C19*2-R
<400> 4
   cccgagggtt gttgatgtcc atc 23
<210> 5
   <211> 35
   <212> DNA
   <213> Artificial Sequence
<220>
   <223> CYP2C19*3-F
<400> 5
   tgatggaaaa attgaatgaa aacatcagga ttgta 35
<210> 6
   <211> 30
   <212> DNA
   <213> Artificial Sequence
<220>
   <223> CYP2C19*3-R
<400> 6
   tcaaaaatgt acttcagggc ttggtcaata 30
<210> 7
   <211> 29
   <212> DNA
   <213> Artificial Sequence
<220>
   <223> CYP2C19*17-F
<220>
   <221> misc_feature
   <222> (21)..(21)
   <223> a or t
<400> 7
   gtttggaagt tgttttgttt wgctaaaac 29
<210> 8
   <211> 22
   <212> DNA
   <213> Artificial Sequence
<220>
   <223> CYP2C19*17-F7
<400> 8
   gcatctctgg ggctgttttc ct 22
<210> 9
   <211> 30
   <212> DNA
   <213> Artificial Sequence
<220>
   <223> CYP2C19*17-F8
<400> 9
   tgtgaagcct gttttatgaa caggatgaat 30
<210> 10
   <211> 30
   <212> DNA
   <213> Artificial Sequence
<220>
   <223> CYP2C19*17-F9
<400> 10
   taatgtgaag cctgttttat gaacaggatg 30
<210> 11
   <211> 26
   <212> DNA
   <213> Artificial Sequence
<220>
   <223> CYP2C19*17-R
<400> 11
   ctgggatttg agctgaggtc ttctga 26
<210> 12
   <211> 21
   <212> DNA
   <213> Artificial Sequence
<220>
   <223> CYP2C19*17-R5
<400> 12
   tcttctgatg cccatcgtgg c 21
<210> 13
   <211> 28
   <212> DNA
   <213> Artificial Sequence
<220>
   <223> probe
<400> 13
   cattgattat ttcccaggaa cccataac 28
<210> 14
   <211> 27
   <212> DNA
   <213> Artificial Sequence
<220>
   <223> probe
<400> 14
   attgattatt tcccaggaac ccataac 27
<210> 15
   <211> 26
   <212> DNA
   <213> Artificial Sequence
<220>
   <223> probe
<400> 15
   ttgattattt cccaggaacc cataac 26
<210> 16
   <211> 25
   <212> DNA
   <213> Artificial Sequence
<220>
   <223> probe
<400> 16
   tgattatttc ccaggaaccc ataac 25
<210> 17
   <211> 24
   <212> DNA
   <213> Artificial Sequence
<220>
   <223> probe
<400> 17
   gattatttcc caggaaccca taac 24
<210> 18
   <211> 23
   <212> DNA
   <213> Artificial Sequence
<220>
   <223> probe
<400> 18
   attatttccc aggaacccat aac 23
<210> 19
   <211> 22
   <212> DNA
   <213> Artificial Sequence
<220>
   <223> probe
<400> 19
   ttatttccca ggaacccata ac 22
<210> 20
   <211> 21
   <212> DNA
   <213> Artificial Sequence
<220>
   <223> probe
<400> 20
   tatttcccag gaacccataa c 21
<210> 21
   <211> 20
   <212> DNA
   <213> Artificial Sequence
<220>
   <223> probe
<400> 21
   atttcccagg aacccataac 20
<210> 22
   <211> 19
   <212> DNA
   <213> Artificial Sequence
<220>
   <223> probe
<400> 22
   tttcccagga acccataac 19
<210> 23
   <211> 18
   <212> DNA
   <213> Artificial Sequence
<220>
   <223> probe
<400> 23
   ttcccaggaa cccataac 18
<210> 24
   <211> 17
   <212> DNA
   <213> Artificial Sequence
<220>
   <223> probe
<400> 24
   tcccaggaac ccataac 17
<210> 25
   <211> 16
   <212> DNA
   <213> Artificial Sequence
<220>
   <223> probe
<400> 25
   cccaggaacc cataac 16
<210> 26
   <211> 25
   <212> DNA
   <213> Artificial Sequence
<220>
   <223> probe
<400> 26
   caccccctga atccaggtaa ggcca 25
<210> 27
   <211> 24
   <212> DNA
   <213> Artificial Sequence
<220>
   <223> probe
<400> 27
   caccccctga atccaggtaa ggcc 24
<210> 28
   <211> 23
   <212> DNA
   <213> Artificial Sequence
<220>
   <223> probe
<400> 28
   caccccctga atccaggtaa ggc 23
<210> 29
   <211> 22
   <212> DNA
   <213> Artificial Sequence
<220>
   <223> probe
<400> 29
   caccccctga atccaggtaa gg 22
<210> 30
   <211> 21
   <212> DNA
   <213> Artificial Sequence
<220>
   <223> probe
<400> 30
   caccccctga atccaggtaa g 21
<210> 31
   <211> 20
   <212> DNA
   <213> Artificial Sequence
<220>
   <223> probe
<400> 31
   caccccctga atccaggtaa 20
<210> 32
   <211> 19
   <212> DNA
   <213> Artificial Sequence
<220>
   <223> probe
<400> 32
   caccccctga atccaggta 19
<210> 33
   <211> 18
   <212> DNA
   <213> Artificial Sequence
<220>
   <223> probe
<400> 33
   caccccctga atccaggt 18
<210> 34
   <211> 19
   <212> DNA
   <213> Artificial Sequence
<220>
   <223> probe
<400> 34
   ccctgaatcc aggtaaggc 19
<210> 35
   <211> 18
   <212> DNA
   <213> Artificial Sequence
<220>
   <223> probe
<400> 35
   ccctgaatcc aggtaagg 18
<210> 36
   <211> 17
   <212> DNA
   <213> Artificial Sequence
<220>
   <223> probe
<400> 36
   ccctgaatcc aggtaag 17
<210> 37
   <211> 16
   <212> DNA
   <213> Artificial Sequence
<220>
   <223> probe
<400> 37
   ccctgaatcc aggtaa 16
<210> 38
   <211> 15
   <212> DNA
   <213> Artificial Sequence
<220>
   <223> probe
<400> 38
   ccctgaatcc aggta 15
<210> 39
   <211> 14
   <212> DNA
   <213> Artificial Sequence
<220>
   <223> probe
<400> 39
   ccctgaatcc aggt 14
<210> 40
   <211> 19
   <212> DNA
   <213> Artificial Sequence
<220>
   <223> probe
<400> 40
   cagagatact ttgagaaca 19
<210> 41
   <211> 20
   <212> DNA
   <213> Artificial Sequence
<220>
   <223> probe
<400> 41
   cagagatact ttgagaacag 20
<210> 42
   <211> 21
   <212> DNA
   <213> Artificial Sequence
<220>
   <223> probe
<400> 42
   cagagatact ttgagaacag a 21
<210> 43
   <211> 22
   <212> DNA
   <213> Artificial Sequence
<220>
   <223> probe
<400> 43
   cagagatact ttgagaacag aa 22
<210> 44
   <211> 23
   <212> DNA
   <213> Artificial Sequence
<220>
   <223> probe
<400> 44
   cagagatact ttgagaacag aag 23
<210> 45
   <211> 24
   <212> DNA
   <213> Artificial Sequence
<220>
   <223> probe
<400> 45
   cagagatact ttgagaacag aaga 24
<210> 46
   <211> 25
   <212> DNA
   <213> Artificial Sequence
<220>
   <223> probe
<400> 46
   cagagatact ttgagaacag aagac 25
<210> 47
   <211> 26
   <212> DNA
   <213> Artificial Sequence
<220>
   <223> probe
<400> 47
   cagagatact ttgagaacag aagaca 26
<210> 48
   <211> 27
   <212> DNA
   <213> Artificial Sequence
<220>
   <223> probe
<400> 48
   cagagatact ttgagaacag aagacac 27
<210> 49
   <211> 28
   <212> DNA
   <213> Artificial Sequence
<220>
   <223> probe
<400> 49
   cagagatact ttgagaacag aagacaca 28
<210> 50
   <211> 29
   <212> DNA
   <213> Artificial Sequence
<220>
   <223> probe
<400> 50
   cagagatact ttgagaacag aagacacaa 29
<210> 51
   <211> 19
   <212> DNA
   <213> Artificial Sequence
<220>
   <223> probe
<400> 51
   tcagagatac tttgagaac 19
<210> 52
   <211> 20
   <212> DNA
   <213> Artificial Sequence
<220>
   <223> probe
<400> 52
   atcagagata ctttgagaac 20
<210> 53
   <211> 21
   <212> DNA
   <213> Artificial Sequence
<220>
   <223> probe
<400> 53
   catcagagat actttgagaa c 21
<210> 54
   <211> 22
   <212> DNA
   <213> Artificial Sequence
<220>
   <223> probe
<400> 54
   acatcagaga tactttgaga ac 22
<210> 55
   <211> 23
   <212> DNA
   <213> Artificial Sequence
<220>
   <223> probe
<400> 55
   tacatcagag atactttgag aac 23
<210> 56
   <211> 24
   <212> DNA
   <213> Artificial Sequence
<220>
   <223> probe
<400> 56
   ttacatcaga gatactttga gaac 24
<210> 57
   <211> 25
   <212> DNA
   <213> Artificial Sequence
<220>
   <223> probe
<400> 57
   cttacatcag agatactttg agaac 25
<210> 58
   <211> 26
   <212> DNA
   <213> Artificial Sequence
<220>
   <223> probe
<400> 58
   tcttacatca gagatacttt gagaac 26
<210> 59
   <211> 27
   <212> DNA
   <213> Artificial Sequence
<220>
   <223> probe
<400> 59
   ctcttacatc agagatactt tgagaac 27
<210> 60
   <211> 28
   <212> DNA
   <213> Artificial Sequence
<220>
   <223> probe
<400> 60
   tctcttacat cagagatact ttgagaac 28
<210> 61
   <211> 29
   <212> DNA
   <213> Artificial Sequence
<220>
   <223> probe
<400> 61
   atctcttaca tcagagatac tttgagaac 29
<210> 62
   <211> 30
   <212> DNA
   <213> Artificial Sequence
<220>
   <223> probe
<400> 62
   tatctcttac atcagagata ctttgagaac 30
<210> 63
   <211> 26
   <212> DNA
   <213> Artificial Sequence
<220>
   <223> probe
<400> 63
   tcagagatac tttgagaaca gaagac 26
<210> 64
   <211> 24
   <212> DNA
   <213> Artificial Sequence
<220>
   <223> probe
<400> 64
   agagatactt tgagaacaga agac 24
<210> 65
   <211> 23
   <212> DNA
   <213> Artificial Sequence
<220>
   <223> probe
<400> 65
   gagatacttt gagaacagaa gac 23
<210> 66
   <211> 22
   <212> DNA
   <213> Artificial Sequence
<220>
   <223> probe
<400> 66
   agatactttg agaacagaag ac 22
<210> 67
   <211> 21
   <212> DNA
   <213> Artificial Sequence
<220>
   <223> probe
<400> 67
   gatactttga gaacagaaga c 21
<210> 68
   <211> 20
   <212> DNA
   <213> Artificial Sequence
<220>
   <223> probe
<400> 68
   atactttgag aacagaagac 20
<210> 69
   <211> 19
   <212> DNA
   <213> Artificial Sequence
<220>
   <223> probe
<400> 69
   tactttgaga acagaagac 19

## Description

### BACKGROUND

### Technical Field

The present invention relates to a kit for amplifying the CYP2C19 gene, a gene amplification method, a polymorphism analysis method, and a drug efficacy evaluation method.

### Related Art

Cytochrome P450 is an enzyme group that is classified as a superfamily and includes a number of subfamilies (for example, CYP1A, CYP1B, CYP2C, CYP2D, CYP2E and CYP3A). Particularly, CYP2C19, that is an isozyme of human CYP2C subfamily, is one of the representative enzymes involved in drug metabolism.

It is known that gene polymorphisms such as CYP2C19*2, CYP2C19*3 and CYP2C19*17 occur in CYP2C19. CYP2C19*2 is a mutation in which guanine at the 681st base of exon 5 is replaced by adenine and causes abnormal splicing, as a result of which the translation initiation site of the genetic information is modified. CYP2C19*3 is a mutation in which guanine at the 636th base of exon 4 is replaced by adenine, and the resulting change to a stop codon causes interruption of translation at the site of this mutation. The gene polymorphisms CYP2C19*2 and CYP2C19*3 both lead to loss or reduction of the enzymatic activity of CYP2C19. CYP2C19* 17 is a mutation in which cytosine at the -806th base of the promoter region is replaced by thymine and causes an increase in the expression and enzymatic activity of CYP2C19.

All of these gene polymorphisms affect the in-blood dynamics and efficacies of drugs. Therefore, analysis of the gene polymorphisms in CYP2C19 is extremely important for estimating the side effects of a drug and determining the drug administration conditions that yield more favorable effects.

Meanwhile, as a method of analyzing the causes of various diseases, the disease liability (susceptibility to diseases) of an individual, the differences in drug efficacy between individuals and the like at the genetic level, detection of point mutation so-called "single-nucleotide polymorphism (SNP)" is widely performed. Examples of a common method of detecting a point mutation include (1) a direct sequencing method in which a region corresponding to a sequence to be detected in a target DNA of a sample is amplified by PCR (polymerase chain reaction) and the entire gene sequence of the resulting amplification product is analyzed, (2) an RFLP (Restriction Fragment Length Polymorphisms) analysis in which a region corresponding to a sequence to be detected in a target DNA of a sample is amplified by PCR and the resulting amplification product is then cleaved by a restriction enzyme that exhibits different cleaving actions depending on the presence or absence of a target mutation in the sequence to be detected, followed by typing by electrophoresis, and (3) an ASP-PCR (Allele Specific Primer PCR) method in which PCR is performed using a primer having a target mutation in its 3'-terminal region and the mutation is evaluated based on the presence or absence of amplification.

However, these methods require, for example, purification of DNA extracted from a sample, electrophoresis and treatment with a restriction enzyme and are, therefore, laborious and costly. In addition, since a reaction vessel needs to be opened once after PCR, the amplification product may potentially contaminate the subsequent reaction system and the analysis accuracy may thereby be reduced. Moreover, since automation of these methods is difficult, these methods are not capable of analyzing a large number of samples. Furthermore, the (3) ASP-PCR method has low specificity, which is also a problem.

In view of these problems, as a point mutation detection method, a method of analyzing the melting temperature (Tm) of a double-stranded nucleic acid formed by a target nucleic acid and a probe has been recently put into practice. Such a method is referred to as, for example, "Tm analysis" or "melting curve analysis". This method is described as follows. That is, first, using a probe that is complementary to a sequence to be detected containing a point mutation as a detection object, a hybrid (double-stranded DNA) is formed between a target single-stranded DNA contained in a detection sample and the probe. Subsequently, the thus formed hybrid is heat-treated, and dissociation (melting) of the hybrid associated with temperature increase is detected based on a change in signal such as absorbance. Then, the Tm value is determined based on the detection results, and the presence or absence of the point mutation is thereby determined. The higher the homology of the hybrid, the larger is the Tm value, and the lower the homology, the smaller is the Tm value. Accordingly, in cases where the Tm value (reference value for evaluation) has been determined in advance for a hybrid formed between a sequence to be detected containing a point mutation and a probe complementary thereto and the Tm value (measurement value) is measured for a hybrid formed between a target single-stranded DNA in a detection sample and the probe, when the measured value and the reference value are the same, it can be determined that the target DNA and the probe are a match, that is, the point mutation is present in the target DNA. On the other hand, when the measured value is smaller than the reference value, it can be determined that the target DNA and the probe are a mismatch, that is, the point mutation is absent in the target DNA. Furthermore, this method can be automated.

### SUMMARY OF INVENTION

### Problems to be Solved by the Invention

However, such a detection method utilizing Tm analysis as described above also has a problem in that a region containing a site to be detected must be specifically and efficiently amplifiable by PCR. Particularly, cytochrome P450 is an enzyme group classified as a superfamily as described above, and the molecular species belonging to the same subfamily (CYP2C subfamily) include isozymes having very high homology and their coding sequences are also extremely similar. Thus, in PCR, even the coding genes of such isozymes other than CYP2C19 may be amplified as well. Moreover, amplification of the coding genes of other isozymes in this manner may lead to, for example, a decrease in the reliability of the results obtained by an analysis of gene polymorphism of CYP2C19.

Under these circumstances, the present applicant has proposed a primer set for specifically and efficiently amplifying regions containing a site of gene polymorphism CYP2C19*2 or CYP2C19*3, respectively (see, for example, WO2008/066162). By using the primer set described in WO2008/066162, regions containing a site of gene polymorphism CYP2C19*2 or CYP2C19*3, respectively, can be simultaneously and specifically amplified in a single reaction solution.

By the way, the majority of the CYP2C19 gene polymorphisms found in Asian population including Japanese are CYP2C19*2 and CYP2C19*3, with the frequency of CYP2C19*17 being low. On the other hand, in some races, CYP2C19*17 is known to occur at a high frequency. For instance, it has been reported that the frequency of CYP2C19*17 is 18% for Swedes and Ethiopians, 25% for Germans and 27% for Poles.

Because of the increasing racial diversification in Japan in recent years, in order to be able to cope with diverse races, it is considered desirable to be capable of simultaneously and specifically amplifying regions containing a site of gene polymorphism CYP2C19*2, CYP2C19*3 or CYP2C19*17, respectively, in a single reaction solution.

However, in order to simultaneously and specifically amplify regions containing a site of gene polymorphism CYP2C19*2, CYP2C19*3 or CYP2C19*17, respectively, in a single reaction solution, it is necessary to search for a set of primers capable of performing amplification under the same temperature condition without inhibiting amplification of the respective regions. Moreover, in the resulting amplification products, a genotype has to be stably detectable using a probe. Therefore, such a primer set is yet to be proposed.

The description provides a primer set for the CYP2C19 gene amplification, which is used for specifically and efficiently amplifying regions containing a site of gene polymorphism CYP2C19*2, CYP2C19*3 or CYP2C19*17, respectively, and a kit for the CYP2C19 gene amplification that includes the primer set. Also disclosed is a gene amplification method using the primer set for the CYP2C19 gene amplification, a polymorphism analysis method, and a drug efficacy evaluation method. The description further provides use of said primer set or kit to amplify target regions in the CYP2C19 gene, to analyze polymorphisms in the CYP2C19 gene or to evaluate drug efficacy.

The invention is defined by the appended claims.

The invention relates to a kit suitable for amplifying target regions of the CYP2C19 gene, the kit comprising the herein below i) primer set and ii) a probe that is capable of hybridizing to a region containing a site of gene polymorphism CYP2C19*2, a probe that is capable of hybridizing to a region containing a site of gene polymorphism CYP2C19*3, and a probe that is capable of hybridizing to a region containing a site of gene polymorphism CYP2C19*17.
[1] A primer set configured to amplify target regions of a CYP2C19 gene by a nucleic acid amplification method, the primer set comprising a primer set (1), a primer set (2), and a primer set (3) that is selected from the group consisting of primer sets (3a), (3b) and (3c),
   the primer set (1) comprising a forward primer that is an oligonucleotide consisting of a base sequence represented by SEQ ID NO:3, and a reverse primer that is an oligonucleotide consisting of a base sequence represented by SEQ ID NO:4,
   the primer set (2) comprising a forward primer that is an oligonucleotide consisting of a base sequence represented by SEQ ID NO:5, and a reverse primer that is an oligonucleotide consisting of a base sequence represented by SEQ ID NO:6,
   the primer set (3a) comprising a forward primer that is an oligonucleotide consisting of a base sequence represented by SEQ ID NO:7, and a reverse primer that is an oligonucleotide consisting of a base sequence represented by SEQ ID NO:11,
   the primer set (3b) comprising a forward primer that is an oligonucleotide consisting of a base sequence represented by SEQ ID NO:7, and a reverse primer that is an oligonucleotide consisting of a base sequence represented by SEQ ID NO:12, and
   the primer set (3c) comprising a forward primer that is an oligonucleotide consisting of a base sequence represented by SEQ ID NO:9, and a reverse primer that is an oligonucleotide consisting of a base sequence represented by SEQ ID NO:11.
[2] The primer set according to [1], wherein the primer set comprises the primer sets (1), (2) and (3b).
[3] The primer set according to [1] or [2], wherein the primer set is configured to amplify the CYP2C19 gene in a biological sample.
[4] The primer set according to [3], wherein the biological sample is whole blood.
[5] A gene amplification method, comprising amplifying target regions in the CYP2C19 gene in a single reaction solution by using a nucleic acid contained in a sample as a template and the herein above defined kit.
[6] The method according to [5], wherein the ratio of the total molar amount of the primer set (1) to the total molar amount of the primer set (2) (primer set (1): primer set (2)) is from 1:1 to 1:3, and the ratio of the total molar amount of the primer set (1) to the total molar amount of the primer set (3) (primer set (1): primer set (3)) is from 1:1.5 to 1:4.
[7] A polymorphism analysis method, comprising:
   amplifying target regions of the CYP2C19 gene by the gene amplification method according to [5] or [6];
   forming hybrids between single-stranded amplified nucleic acids that are amplification products obtained by the amplification and probes, the probes including a probe that is capable of hybridizing to a region containing a site of gene polymorphism CYP2C19*2, a probe that is capable of hybridizing to a region containing a site of gene polymorphism CYP2C19*3, and a probe that is capable of hybridizing to a region containing a site of gene polymorphism CYP2C19*17;
   changing the temperature of a reaction solution containing the hybrids and measuring changes in signals reflecting dissociation of the hybrids; and
   analyzing polymorphisms of the CYP2C19 gene based on the changes in the signals.
[8] The method according to [7], wherein the amplifying of the target regions and the forming of hybrids proceed simultaneously.
[9] The method according to [7] or [8], wherein the probes are fluorescently labeled probes.
[10] A drug efficacy evaluation method comprising:
   analyzing polymorphisms of the CYP2C19 gene by the polymorphism analysis method according to any one of [7] to [9]; and
   evaluating the efficacy of a drug based on a result of the analysing.
[13] The kit according to [11] or [12], wherein the probes are fluorescently labeled probes.
[14] Use of the herein above recited kit to analyze polymorphisms in the CYP2C19 gene or to evaluate drug efficacy.

### BRIEF DESCRIPTION OF THE DRAWINGS

FIGs. 1A to 1C show the amplification evaluation values obtained in Experimental Example 1 for target regions containing a site of gene polymorphism CYP2C19*17, CYP2C19*2 or CYP2C19*3;
FIGs. 2A to 2C show the amplification evaluation values obtained in Experimental Example 2 for target regions containing a site of gene polymorphism CYP2C19*17, CYP2C19*2 or CYP2C19*3;
FIGs. 3A to 3C show the amplification evaluation values obtained in Experimental Example 3 for target regions containing a site of gene polymorphism CYP2C19*17, CYP2C19*2 or CYP2C19*3; and
FIGs. 4A to 4E show the results of Tm analysis performed in Experimental Example 4.

### DETAILED DESCRIPTION OF THE INVENTION

### Mode for Carrying Out the Invention

The mode for carrying out the invention will now be described.

As used herein, the notations "from ... to ..." or "to" expressing a numerical range indicate a range including the numerical values before and after "to", as the minimum value and the maximum value, respectively.

As regard to the amount of a component of a composition, when multiple substances corresponding to the same component exist in the composition, the amount of the component used herein refers to the total amount of the multiple substances in the composition unless otherwise specified.

The term "step" as used herein includes not only a separate step but also a step that is not clearly distinguished from other steps as long as the desired effect of the step is obtained therefrom.

The term "Tm value" used herein refers to the temperature at which a double-stranded nucleic acid dissociates (dissociation temperature: Tm) and is generally defined as the temperature at which the absorbance at 260 nm has increased by 50% of the total increase in absorbance resulting from complete dissociation of the double-stranded nucleic acid. When a solution containing a double-stranded nucleic acid such as a double-stranded DNA is heated, the absorbance at 260 nm of the double-stranded nucleic acid increases. This is because the hydrogen bonds between the strands of the double-stranded DNA are broken by the heating, as a result of which the double-stranded DNA is dissociated into single-stranded DNAs (melting of DNA). When the double-stranded DNA is completely dissociated into single-stranded DNAs, the single-stranded DNAs exhibit an absorbance that is about 1.5 times as high as the absorbance at the time of the initiation of the heating (i.e., the absorbance of a solution in which all DNAs are in the form of the double-stranded DNA), which indicates that the dissociation has been completed. The Tm value is defined based on this phenomenon.

### Primer Set for Amplifying CYP2C19 Gene

The primer set of the present disclosure configured to amplify target regions of a CYP2C19 gene by a nucleic acid amplification method, which may be hereinafter simply referred to as "the primer set of the present disclosure", includes a primer set (1), a primer set (2), and a primer set (3) that is selected from the group consisting of primer sets (3a), (3b) and (3c), and is used to amplify target regions of a CYP2C19 gene by a nucleic acid amplification method.
Primer set (1) includes a forward primer that is an oligonucleotide consisting of a base sequence represented by SEQ ID NO:3, and a reverse primer that is an oligonucleotide consisting of a base sequence represented by SEQ ID NO:4.
Primer set (2) includes a forward primer that is an oligonucleotide consisting of a base sequence represented by SEQ ID NO:5, and a reverse primer that is an oligonucleotide consisting of a base sequence represented by SEQ ID NO:6.
Primer set (3a) includes a forward primer that is an oligonucleotide consisting of a base sequence represented by SEQ ID NO:7, and a reverse primer that is an oligonucleotide consisting of a base sequence represented by SEQ ID NO:11.
Primer set (3b) includes a forward primer that is an oligonucleotide consisting of a base sequence represented by SEQ ID NO:7, and a reverse primer that is an oligonucleotide consisting of a base sequence represented by SEQ ID NO:12.
Primer set (3c) includes a forward primer that is an oligonucleotide consisting of a base sequence represented by SEQ ID NO:9, and a reverse primer that is an oligonucleotide consisting of a base sequence represented by SEQ ID NO:11.

As described below in detail, the primer sets (1) and (2) are primer sets for amplifying regions containing sites of gene polymorphisms CYP2C19*2 and CYP2C19*3, respectively. Further, the primer sets (3a), (3b) and (3c) are all primer sets for amplifying a region containing a site of gene polymorphism CYP2C19*17.

By using the primer sets of the embodiment, regions containing a site of gene polymorphism CYP2C19*2, CYP2C19*3 or CYP2C19*17, respectively, can be simultaneously and specifically amplified in a single reaction solution. This enables a reduction in labor and cost as compared to conventional methods. In addition, since three target regions are specifically amplified in a single reaction solution in this manner, for example, by using probes that are each complementary to the sequence to be detected in the respective target regions, the reaction solution can be directly used to perform Tm analysis and to determine the types of the above-described three kinds of polymorphism.

The primer set (1) is, as described above, a set of a pair of primers that includes a forward primer which is an oligonucleotide having the base sequence represented by SEQ ID NO:3 and a reverse primer which is an oligonucleotide having the base sequence represented by SEQ ID NO:4.

SEQ ID NO:1 is the base sequence of a genomic DNA that includes exons 4 and 5 of the CYP2C19 gene in human chromosome 10. In SEQ ID NO:1, the region of the 1st to 161st bases represents exon 4, the region of the 1323rd to 1449th bases represents exon 5, and the region therebetween represents an intron.

The oligonucleotide having the base sequence represented by SEQ ID NO:3 is an oligonucleotide having a sequence identical to the region of the 1302nd to 1341st bases of SEQ ID NO:1. The 1341st base (adenine) corresponds to the 3'-end of the oligonucleotide.

The oligonucleotide having the base sequence represented by SEQ ID NO:4 is an oligonucleotide having a sequence complementary to the region of the 1442nd to 1464th bases of SEQ ID NO:1. Cytosine, which is complementary to the 1442nd base (guanine), corresponds to the 3'-end of the oligonucleotide.

That is, the primer set (1) is a primer set for amplifying the region of the 1302nd to 1464th bases of SEQ ID NO:1 and a complementary strand thereof.

The 1361st base of SEQ ID NO:1 corresponds to the 681st base of exon 5. A point mutation (681G, 681A) that affects the function of CYP2C19 is known to occur at the 681st base of exon 5, and this polymorphism is the above-described CYP2C19*2. The polymorphism of this site can be represented as "681G/G" or "681A/A" in the case of a homozygote, or "681G/A" in the case of a heterozygote.

The primer set (2) is, as described above, a set of a pair of primers that includes a forward primer which is an oligonucleotide having the base sequence represented by SEQ ID NO:5 and a reverse primer which is an oligonucleotide having the base sequence represented by SEQ ID NO:6.

The oligonucleotide having the base sequence represented by SEQ ID NO:5 is an oligonucleotide having a sequence identical to the region of the 109th to 143rd bases of SEQ ID NO:1. The 143rd base (adenine) corresponds to the 3'-end of the oligonucleotide.

The oligonucleotide having the base sequence represented by SEQ ID NO:6 is an oligonucleotide having a sequence complementary to the region of the 231st to 260th bases of SEQ ID NO:1. Adenine, which is complementary to the 231st base (thymine), corresponds to the 3'-end of the oligonucleotide.

That is, the primer set (2) is a primer set for amplifying the region of the 109th to 260th bases of SEQ ID NO:1 and a complementary strand thereof.

The 155th base of SEQ ID NO:1 corresponds to the 636th base of exon 4. A point mutation (636G, 636A) that affects the function of CYP2C19 is known to occur at the 636th base of exon 4, and this polymorphism is the above-described CYP2C19*3. The polymorphism of this site can be represented as "636G/G" or "636A/A" in the case of a homozygote, or "636G/A" in the case of a heterozygote.

The primer set (3a) is, as described above, a set of a pair of primers that includes a forward primer which is an oligonucleotide having the base sequence represented by SEQ ID NO:7 and a reverse primer which is an oligonucleotide having the base sequence represented by SEQ ID NO:11.

SEQ ID NO:2 is a base sequence in the 5'-upstream of the coding region of the CYP2C19 gene in human chromosome 10.

Among oligonucleotides having the base sequence represented by SEQ ID NO:7, the one in which the 21st base from the 5'-end side is thymine is an oligonucleotide having a sequence identical to the region of the 4113rd to 4141st bases of SEQ ID NO:2. The 4141st base (cytosine) corresponds to the 3'-end of the oligonucleotide.

The oligonucleotide having the base sequence represented by SEQ ID NO:11 is an oligonucleotide having a sequence complementary to the region of the 4232nd to 4257th bases of SEQ ID NO:2. Adenine, which is complementary to the 4232nd base (thymine), corresponds to the 3'-end of the oligonucleotide.

That is, the primer set (3a) is a primer set for amplifying the region of the 4113rd to 4257th bases of SEQ ID NO:2 and a complementary strand thereof.

The 21st base from the 5'-end side of SEQ ID NO:7 corresponds to the 4133rd base of SEQ ID NO:2, and it has been presumed that this base is mutated to adenine in about 0.09% of the world population.

The primer set (3b) is, as described above, a set of a pair of primers that includes a forward primer which is an oligonucleotide having the base sequence represented by SEQ ID NO:7 and a reverse primer which is an oligonucleotide having the base sequence represented by SEQ ID NO:12.

The oligonucleotide having the base sequence represented by SEQ ID NO:12 is an oligonucleotide having a sequence complementary to the region of the 4219th to 4239th bases of SEQ ID NO:2. Cytosine, which is complementary to the 4219th base (guanine), corresponds to the 3'-end of the oligonucleotide. That is, the primer set (3b) is a primer set for amplifying the region of the 4113rd to 4239th bases of SEQ ID NO:2 and a complementary strand thereof.

The primer set (3c) is, as described above, a set of a pair of primers that includes a forward primer which is an oligonucleotide having the base sequence represented by SEQ ID NO:9 and a reverse primer which is an oligonucleotide having the base sequence represented by SEQ ID NO:11.

The oligonucleotide having the base sequence represented by SEQ ID NO:9 is an oligonucleotide having a sequence identical to the region of the 4058th to 4087th bases of SEQ ID NO:2. The 4087th base (thymine) corresponds to the 3'-end of the oligonucleotide. That is, the primer set (3c) is a primer set for amplifying the region of the 4058th to 4257th bases of SEQ ID NO:2 and a complementary strand thereof.

The 4195th base (the 806th base from the 3'-end side) of SEQ ID NO:2 corresponds to the -806th base in the 5'-upstream of the coding region. A point mutation (-806C, -806T) that affects the function of CYP2C19 is known to occur at this -806th base, and this polymorphism is the above-described CYP2C19*17. The polymorphism of this site can be represented as "-806C/C" or "-806T/T" in the case of a homozygote, or "-806C/T" in the case of a heterozygote.

The primer set of the present disclosure may include any of the primer sets (3a), (3b) and (3c) as the primer set (3); however, from the standpoints of efficiency of amplification by PCR, the tolerability against changes in the reaction temperature and the like, it is preferable that the primer set of the present disclosure include the primer set (3b).

At least one oligonucleotide included in the primer set of the present disclosure may be a modified oligonucleotide having the same base sequence as the above-described respective base sequence, except that 1 to 3 bases are extended, shortened, inserted, deleted or substituted. That is, the primer set of the present disclosure also encompasses those primer sets in which at least one oligonucleotide is a modified oligonucleotide. Even when at least one oligonucleotide included in the primer set of the present disclosure is a modified oligonucleotide, regions containing a site of gene polymorphism CYP2C19*2, CYP2C19*3 or CYP2C19*17, respectively, can be simultaneously and specifically amplified in a single reaction solution.

It is preferable that the primer set of the present disclosure be used for amplifying a target region of the CYP2C19 gene contained in a biological sample such as a whole blood sample. The invention also extends to use of said primer set to amplify target regions in the CYP2C19 gene, to analyze polymorphisms in the CYP2C19 gene or to evaluate drug efficacy.

### Gene Amplification Method

The gene amplification method of the present disclosure includes at least amplifying target regions in the CYP2C19 gene in a single reaction solution by using a nucleic acid contained in a sample as a template and the primer set. According to the gene amplification method of the present disclosure, it is possible to simultaneously and specifically amplify regions containing a site of gene polymorphism CYP2C19*2, CYP2C19*3 or CYP2C19*17 respectively in a single reaction solution.

Examples of a sample containing a template nucleic acid include a biological sample. Examples of the biological sample include: whole blood; oral cells such as oral mucosa; somatic cells of nails, hairs and the like; germ cells; sputum; amniotic fluid; paraffin-embedded tissues; urine; gastric juice; gastric lavage fluid; and suspensions thereof. Further, a nucleic acid isolated from a biological sample may also be used as a template. For example, for isolation of a genomic DNA from whole blood, a commercially available genomic DNA isolation kit can be employed. Alternatively, an amplification product obtained by amplifying DNA contained in a biological sample by a gene amplification method may also be used as a template. Or, an amplification product which is obtained by generating a cDNA from an RNA contained in a biological sample by reverse transcription PCR and amplifying the cDNA by a gene amplification method may also be used as a template. Preferably the sample is from a human.

The nucleic acid amplification method used in the amplification step is not particularly restricted. Examples thereof include a PCR method, Nucleic Acid Sequence-Based Amplification (NASBA) method, Transcription-Mediated Amplification (TMA) method and Strand Displacement Amplification (SDA) method, among which a PCR method is preferable.

In the amplification step, the ratio of the amount of a sample to be added to a reaction solution to the amount of the reaction solution (that is, the addition amount of the sample with respect to the amount of the reaction solution) is not particularly restricted. Specifically, when the sample is a biological sample (e.g., a whole blood sample), the lower limit of the addition amount of the sample with respect to the amount of the reaction solution is preferably 0.01% by volume or higher, more preferably 0.05% by volume or higher, still more preferably 0.1% by volume or higher. Further, when the sample is a biological sample (e.g., a whole blood sample), the upper limit of the ratio is preferably 2% by volume or lower, more preferably 1% by volume or lower, still more preferably 0.5% by volume or lower.

In the polymorphism analysis method described below, for example, in cases where optical detection is performed using a labeled probe, it is preferable that the ratio of a biological sample to be added to the reaction solution (that is, the addition amount of the sample with respect to the amount of the reaction solution) be set at, for example, from 0.1% by volume to 0.5% by volume. By setting the ratio within this range, for example, influences of the generation of a precipitate or the like due to denaturation may be sufficiently inhibited and the measurement accuracy of an optical method may thus be improved. In addition, since inhibition of PCR by contaminants in the biological sample may be sufficiently suppressed thereby, it may be expected that the amplification efficiency is further improved.

It is preferable that albumin be further added to the reaction solution prior to the start of the amplification reaction. By such addition of albumin, for example, not only the effects of the generation of a precipitate or turbidity may be further reduced but also the amplification efficiency may be further improved.

The ratio of the albumin to be added to the reaction solution (that is, the addition amount of the albumin with respect to the amount of the reaction solution) is, for example, 0.01% by mass to 2% by mass, preferably 0.1% by mass to 1% by mass, more preferably 0.2% by mass to 0.8% by mass. Examples of the albumin include, but are not particularly limited to, bovine serum albumin (BSA), human serum albumin, rat serum albumin and horse serum albumin. These albumins may be used singly, or in combination of two or more thereof.

The amplification performed in the amplification step will now be described referring to a PCR method as an example; however, the invention is not restricted thereto.

First, a PCR solution containing a template nucleic acid and the primer set of the present disclosure is prepared.

The ratio of each primer to be added to the PCR solution (that is, the addition amount of the each primer with respect to the amount of the PCR solution) is not particularly restricted. For example, the total amount of the forward and reverse primers of the primer set (1) to be added is preferably from 1.0 mol/L to 2.0 µmol/L, and more preferably 1.4 µmol/L to 1.6 µmol/L, with respect to the amount of the PCR solution.

The total amount of the forward and reverse primers of the primer set (2) to be added is preferably from 2.5 µmol/L to 3.5 µmol/L, and more preferably 2.8 µmol/L to 3.2 µmol/L, with respect to the amount of the PCR solution.

The total amount of the forward and reverse primers of the primer set (3) to be added is preferably from 3.0 µmol/L to 5.0 µmol/L, and more preferably from 3.8 µmol/L to 4.2 µmol/L, with respect to the amount of the PCR solution.

The content ratio of the primer sets (1), (2) and (3) in the PCR solution is not particularly restricted. From the standpoint of amplification efficiency, the ratio of the total molar amount of the primer set (1) to that of the primer set (2) (primer set (1): primer set (2)) is preferably from 1:1 to 1:3, and more preferably from 1:1.5 to 1:2.5. The ratio of the total molar amount of the primer set (1) to that of the primer set (3) (primer set (1): primer set (3)) is preferably from 1:1.5 to 1:4, and more preferably from 1:2 to 1:3.

The molar ratio between the forward primer (F) and the reverse primer (R) (F:R) in each of the primer sets (1), (2) and (3) is not particularly restricted. This molar ratio is, for example, preferably F:R = 1:0.25 to 1:4.

Other composition components in the PCR solution are not particularly restricted, and conventionally known components may be employed without being particularly restricted in terms of amounts thereof. Examples of other composition components include a DNA polymerase, a nucleotide such as nucleoside triphosphate (dNTP), and a solvent. In the PCR solution, the order of adding these composition components is not restricted at all.

The DNA polymerase is not particularly restricted. For example, a conventionally known thermoduric bacteria-derived polymerase can be used. Specific examples thereof include *Thermus aquaticus*-derived DNA polymerase (see U.S. Patent No. 4,889,818 and U.S. Patent No. 5,079,352 (trade name: Taq polymerase), *Thermus thermophilus*-derived DNA polymerase (see WO91/09950) (rTth DNA polymerase), *Pyrococcus furiosus*-derived DNA polymerase (see WO92/09689) (Pfu DNA polymerase; manufactured by Strategene Corp.) and *Thermococcus litoralis-*derived DNA polymerase (see European Patent No. 0455430) (VENT (trademark); manufactured by New England Biolabs, Inc.), all of which are commercially available. Thereamong, the *Thermus aquaticus*-derived heat-resistant DNA polymerase is preferable.

The DNA polymerase may be added to the PCR solution at any amount that is usually used in the art for the purpose of amplifying a target nucleic acid.

Examples of the nucleoside triphosphate generally include dNTPs (e.g., dATP, dGTP, dCTP, dTTP and dUTP). A dNTP may be added to the PCR solution at any amount that is usually used in the art for the purpose of amplifying a target nucleic acid.

Examples of the solvent include buffers such as Tris-HCl, Tricine, 2-morpholinoethanesulfonic acid (MES), 3-morpholinopropanesulfonic acid (MOPS), 4-(2-hydroxyethyl)-1-piperazineethanesulfonic acid (HEPES) and *N*-cyclohexyl-3-aminopropanesulfonic acid (CAPS) and, for example, a commercially available PCR buffer or a buffer included in a PCR kit may be used as is.

The PCR solution may further contain glycerol, heparin, betaine, NaN₃, KCl, MgCl₂, MgSO₄, and/or the like.

PCR usually includes the following three steps: (i) dissociation of a double-stranded nucleic acid into single-stranded nucleic acids (dissociation step); (ii) annealing of primers to a template nucleic acid (annealing step); and (iii) extension of a nucleic acid sequence from the primers using DNA polymerase (extension step). The conditions of each step are not particularly restricted. As for the conditions of the dissociation step, for example, the dissociation step is carried out preferably at 90°C to 99°C for 1 second to 120 seconds, more preferably at 92°C to 95°C for 1 second to 60 seconds. As for the conditions of the annealing step, for example, the annealing step is carried out preferably at 40°C to 70°C for 1 second to 300 seconds, more preferably at 50°C to 70°C for 5 seconds to 60 seconds. Further, as for the conditions of extension step, for example, the extension step is carried out preferably at 50°C to 80°C for 1 second to 300 seconds, more preferably at 50°C to 80°C for 5 seconds to 60 seconds. The number of cycles is also not particularly restricted and it is, for example, preferably not less than 30 cycles, taking the above-described three steps as 1 cycle. The upper limit of the number of cycles is not particularly restricted and it may be in a total of, for example, 100 cycles or less, preferably 70 cycles or less, more preferably 50 cycles or less. The temperature change in each step may be automatically controlled using, for example, a thermal cycler. PCR may also be performed in two steps by carrying out the annealing and extension steps under the same temperature condition.

Amplification products that correspond to three target regions containing a site of gene polymorphism CYP2C19*2, CYP2C19*3 or CYP2C19*17, respectively, can be obtained in the above-described manner.

### Polymorphism Analysis Method

The polymorphism analysis method of the present disclosure includes at least: amplifying target regions of the CYP2C19 gene by the gene amplification method according to [5] or [6]; forming hybrids between single-stranded amplified nucleic acids that are amplification products obtained by the amplification and probes that are capable of hybridizing to a region containing a site of gene polymorphism CYP2C19*2, CYP2C19*3 or CYP2C19*17, respectively; changing the temperature of a reaction solution containing the hybrids and measuring changes in signals reflecting dissociation of the hybrids; and analyzing polymorphisms of the CYP2C19 gene based on the changes in the signals. According to the polymorphism analysis method of the present disclosure, it is possible to simultaneously and specifically amplify regions containing a site of gene polymorphism CYP2C19*2, CYP2C19*3 or CYP2C19*17, respectively, in a single reaction solution to enable polymorphism analysis of the CYP2C19 gene.

Since the above-described amplification step is the same as the amplification step in the gene amplification method of the present disclosure, detailed descriptions thereof are omitted here.

Next, in the hybrid formation step, hybrids are formed between amplified single-stranded nucleic acids obtained as amplification products in the amplification step and probes that are capable of hybridizing to a region containing a site of gene polymorphism CYP2C19*2, CYP2C19*3 or CYP2C19*17, respectively (namely, probes including a probe for CYP2C19*2, that is a probe capable of hybridizing to a region containing a site of gene polymorphism CYP2C19*2, a probe for CYP2C19*3, that is a probe capable of hybridizing to a region containing a site of gene polymorphism CYP2C19*3, and a probe for CYP2C19*17, that is a probe capable of hybridizing to a region containing a site of gene polymorphism CYP2C19*17). Hereinafter, the probe for CYP2C19*2, the probe for CYP2C19*3, and the probe for CYP2C19*17 are also generically referred to as "probes for polymorphism analysis".

The amplified single-stranded nucleic acids can be prepared by, for example, heating the reaction solution and thereby dissociating amplified double-stranded nucleic acids obtained as amplification products in the amplification step. Details of the sequences and the like of the probes for polymorphism analysis will be described below.

The timing of adding the probes for polymorphism analysis to the reaction solution is not particularly restricted. For example, the probes for polymorphism analysis can be added before the amplification step, at the start of the amplification step, in the middle of the amplification step or after the amplification step. Particularly, it is preferable to add the probes for polymorphism analysis before the amplification step or at the start of the amplification step because this enables the amplification reaction and hybridization to be carried out continuously. That is, from the standpoint of processing efficiency, it is preferable that the amplification step and the hybrid formation step proceed simultaneously.

The ratio of the probes for polymorphism analysis added to the reaction solution (that is, the addition amount of the probes with respect to the amount of the reaction solution) is not particularly restricted. For example, the probes for polymorphism analysis are preferably added to the reaction solution so that the addition amount of the probes with respect to the amount of the reaction solution becomes from 10 nmol/L to 400 nmol/L, and more preferably from 20 nmol/L to 200 nmol/L.

The method and conditions for hybridization of the amplified single-stranded nucleic acids and the probes for polymorphism analysis are not particularly restricted. Any conditions that are known in the art for the purposes of dissociating amplified double-stranded nucleic acids into amplified single-stranded nucleic acids and hybridizing the single-stranded nucleic acids with each other can be applied as they are.

For example, the heating temperature for dissociation is not particularly restricted as long as it allows the amplification products to dissociate, and it is, for example, from 85°C to 95°C. The heating time is also not particularly restricted, and it is usually from 1 second to 10 minutes, and preferably from 1 second to 5 minutes. The thus dissociated amplified single-stranded nucleic acids and the probes for polymorphism analysis can be hybridized by, for example, lowering, after the dissociation, the heating temperature used for the dissociation. The temperature condition of the hybridization is, for example, from 40°C to 50°C.

In the amplification step, when the primer set of the present disclosure is made to coexist with the probes for polymorphism analysis, in order to prevent the probes for polymorphism analysis themselves from becoming the reaction targets of DNA polymerase and thus being extended, it is preferable that the fluorescent label described below be added to the 3'-terminal region of the respective probes for polymorphism analysis or a phosphate group be added to the 3'-end of the respective probes for polymorphism analysis.

From the standpoint of detection efficiency, it is preferable that the probes for polymorphism analysis be labeled probes to which a label is attached. Particularly, in the polymorphism analysis method of the present disclosure, since three kinds of probes for polymorphism analysis, which are the probe for CYP2C19*2, the probe for CYP2C19*3 and the probe for CYP2C19*17, are used, it is preferable that these three kinds of probes for polymorphism analysis be labeled with different labels that are each detected under different conditions. The use of different labels in this manner enables each amplification product to be analyzed separately by changing the detection conditions, even in a single reaction solution.

Specific examples of a labeling substance used in the labeled probes include fluorescent dyes and fluorophores. As the labeled probes, fluorescently labeled probes that are each labeled with such a fluorescent dye or fluorophore are preferably used. Specific examples of the fluorescently labeled probes include probes which are labeled with a fluorescent dye, which emit fluorescence when not hybridized to a complementary sequence, and fluorescence of which is reduced (for example, quenched) upon hybrid formation (that is, when the probe hybridizes with a complementary sequence).

Probes utilizing such fluorescence quenching phenomenon are generally referred to as "fluorescence quenching probes". It is preferable that a fluorescence quenching probe be labeled with a fluorescent dye at a base in the 3'-terminal region (e.g., 3'-end) or 5'-terminal region (e.g., 5'-end) of its oligonucleotide and that the base to be labeled is cytosine (C). In this case, it is preferable that the base sequence of each fluorescence quenching probe be designed such that, in the sequence to be detected to which the fluorescence quenching probe hybridizes, the base to be paired with the terminal base C of the fluorescence quenching probe or a base that is apart therefrom by 1 to 3 bases is guanine (G). Such a fluorescence quenching probe is generally referred to as "guanine quenching probe" and known as a so-called "Q Probe (registered trademark)".

When such a guanine quenching probe hybridizes to the sequence to be detected, its terminal cytosine (C) labeled with a fluorescent dye is brought closer to guanine (G) in the sequence to be detected, as a result of which a phenomenon that the emission of the fluorescent dye is weakened (the fluorescence intensity is reduced) occurs. By using such a guanine quenching probe, hybridization and dissociation can be easily verified based on the changes in the signal. The labeling substance can be usually bound to a phosphate group of the respective nucleotides.

In addition to the detection method using Q Probe, a known detection mode may also be adopted. Examples thereof include a Taq-Man probe method, an RFLP method, a hybridization probe method, a molecular beacon method and an MGB probe method.

Examples of the fluorescent dye include, but are not particularly limited to, fluoresceins, phosphors, rhodamines and polymethine dye derivatives. Examples of commercially available fluorescent dyes include PACIFIC BLUE (registered trademark, manufactured by Molecular Probes, Inc.), TAMRA (registered trademark, manufactured by Molecular Probes, Inc.), BODIPY FL (registered trademark, manufactured by Molecular Probes, Inc.), FluorePrime (trade name, manufactured by Amersham Pharmacia Biotech Inc.), Cy3 and Cy5 (trade names, manufactured by Amersham Pharmacia Biotech Inc.), Fluoredite (trade name, manufactured by Millipore Corporation) and FAM (registered trademark, manufactured by ABI). The combination of fluorescent dyes used for the multiple probes is not particularly restricted as long as the fluorescent dyes can be detected under different conditions, and examples thereof include a combination of PACIFIC BLUE (detection wavelength: 450 nm to 480 nm), TAMRA (detection wavelength: 585 nm to 700 nm) and BODIPY FL (detection wavelength: 515 nm to 555 nm).

In cases where the probes for polymorphism analysis are probes that are each labeled with a labeling substance such as a fluorescent dye, unlabeled probes having a sequence identical to that of the respective labeled probes may also be used in combination. This advantageously enables, for example, control of the signal intensity, such as fluorescence intensity, to be detected. A phosphate group may be added to the 3'-end of the unlabeled probes.

Next, in the signal measurement step, the temperature of the reaction solution containing the hybrids is changed and the changes in the signals reflecting dissociation of the respective hybrids are measured. The measurement of the signals indicating the dissociation state of the hybrids may be carried out by measuring the absorbance at 260 nm; however, it is preferably carried out by measuring the signal of each labeling substance. By measuring the signal of each labeling substance, the detection sensitivity can be improved.

Changes in the signals reflecting dissociation of the hybrids are generated by changing the temperature of the reaction solution. For example, the reaction solution is heated, that is, the hybrids formed between the amplified single-stranded nucleic acids and the probes for polymorphism analysis are heated, and changes in the signal associated with temperature increase are measured. As described above, for example, in cases where a probe whose terminal base C is labeled (guanine quenching probe) is used, the fluorescence is reduced (or quenched) in the state where the probe is hybridized with an amplified single-stranded nucleic acid, while the probe emits fluorescence in a dissociated state. Accordingly, for example, an increase in the fluorescence intensity associated with temperature increase can be measured by gradually heating the hybrids showing a reduced (or quenched) fluorescence. When a labeled probe is used, the signal can be measured under conditions that conform to the labeling substance of the labeled probe.

The temperature range in which changes in the signal are measured is not particularly restricted. For example, the measurement is initiated at room temperature to 85°C, preferably 25°C to 70°C, and terminated at 40°C to 105°C. The rate of the temperature increase is also not particularly restricted and it is, for example, from 0.1 °C/sec to 20°C/sec, and preferably from 0.3°C/sec to 5°C/sec.

Next, in the analysis step, the polymorphisms in the CYP2C19 gene are analyzed based on the above-described changes in the signal. In this process, it is preferable that the Tm value be determined by analyzing changes in the signal obtained in the signal measurement step and that the polymorphisms in the CYP2C19 gene be analyzed based on the thus determined Tm value.

For example, the Tm value can be determined as follows. As described above, for example, in cases where a probe whose terminal base C is labeled (guanine quenching probe) is used, the amount of change in fluorescence intensity per unit time at each temperature is calculated from the measured change in fluorescence intensity. When the amount of change is defined as [-d(increase in fluorescence intensity)/dt], for example, the temperature showing the lowest value can be determined as the Tm value. When the amount of change is defined as [d(increase in fluorescence intensity)/dt], for example, the temperature showing the highest value can be determined as the Tm value.

On the other hand, in cases where a probe that does not show a signal by itself but shows a signal when hybridized is used as a labeled probe, the Tm value can be determined by measuring the decrease in fluorescence intensity.

In the analysis step, the polymorphisms in the CYP2C19 gene can be analyzed based on the Tm values determined in this manner.

For example, in cases where a base of the target base site is presumed to be of a mutant-type and a probe for polymorphism analysis that is complementary to the sequence to be detected including the mutant-type base is used, the target base can be determined to be of the mutant-type if the Tm value of the resultant hybrid is the same as the Tm value of a completely complementary hybrid that is predetermined as a reference value. Meanwhile, the target base can be determined to be of normal-type if the Tm value of the resultant hybrid is the same as the Tm value of a hybrid different from the resultant hybrid by a single base (a value smaller than the Tm value of a completely complementary hybrid) that is predetermined as a reference value. When both of such Tm values are detected, it can be determined that a mutant-type nucleic acid and a normal-type nucleic acid coexist.

In the above descriptions, the hybrids are heated and changes in the signal associated with temperature increase are measured in the signal measurement step; however, changes in the signal during hybrid formation may be measured instead. In other words, when the temperature of the reaction solution containing the probes for polymorphism analysis is decreased for hybrid formation, changes in the signal associated with the temperature decrease may be measured.

Specifically, in cases where a labeled probe that shows a signal by itself but no longer shows the signal when hybridized (e.g., a guanine quenching probe) is used, fluorescence is emitted in the state where an amplified single-stranded nucleic acid and the labeled probe are dissociated; however, when they are hybridized due to a decrease in temperature, the fluorescence is reduced (or quenched). Accordingly, for example, the reduction in fluorescence intensity associated with temperature decrease can be measured by gradually lowering the temperature of the reaction solution. Meanwhile, in cases where a labeled probe that does not show a signal by itself but shows the signal when hybridized is used, no fluorescence is emitted in the state where an amplified single-stranded nucleic acid and the labeled probe are dissociated; however, fluorescence is emitted when they are hybridized due to a decrease in temperature. Accordingly, for example, the increase in fluorescence intensity associated with temperature decrease can be measured by gradually lowering the temperature of the reaction solution.

Details of the probes for polymorphism analysis that are used in the polymorphism analysis method of the present disclosure will be described below.

The probes for polymorphism analysis are not particularly restricted and can be designed by a conventionally known method. For example, the probes for polymorphism analysis may each be designed as a sequence to be detected that contains a site of gene polymorphism, based on the sequence of a sense strand or the sequence of an antisense strand of the CYP2C19 gene. Further, the base of the site of gene polymorphism can be determined as appropriate in accordance with the type of the polymorphism.

For example, in the case of CYP2C19*2, since polymorphisms of "G" and "A" are known at the 1361st base of SEQ ID NO:1, it is possible to use, as a probe for CYP2C19*2, a probe that is complementary to either a sequence to be detected in which the 1361st base is G or a sequence to be detected in which the 1361st base is A (a probe for detection of sense strand) and a probe that is complementary to the sequence of an antisense strand thereof (a probe for detection of antisense strand).

In the case of CYP2C19*3, since polymorphisms of "G" and "A" are known at the 155th base of SEQ ID NO:1, it is possible to use, as a probe for CYP2C19*3, for example, a probe that is complementary to either a sequence to be detected in which the 155th base is G or a sequence to be detected in which the 155th base is A (a probe for detection of sense strand) and a probe that is complementary to the sequence of an antisense strand thereof (a probe for detection of antisense strand).

In the case of CYP2C19*17, since polymorphisms of "C" and "T" are known at the 4195th base of SEQ ID NO:2, it is possible to use, as a probe for CYP2C19*17, for example, a probe that is complementary to either a sequence to be detected in which the 4195th base is C or a sequence to be detected in which the 4195th base is T (a probe for detection of sense strand) and a probe that is complementary to the sequence of an antisense strand thereof (a probe for detection of antisense strand).

Specific examples of the probe for CYP2C19*2, probe for CYP2C19*3 and probe for CYP2C19*17 are shown in Tables 1 to 3, respectively. The probes shown in Table 1 are examples of the probe for CYP2C19*2 and they are probes for detecting an antisense strand. The probes shown in Table 2 are examples of the probe for CYP2C19*3 and they are also probes for detecting an antisense strand. The probes shown in Table 3 are examples of the probe for CYP2C19*17 and they are probes for detecting a sense strand.

In Tables 1 to 3, the capitalized bases each indicate the base of the site of gene polymorphism CYP2C19*2, CYP2C19*3 or CYP2C19*17. The capitalized bases can be replaced with "r", which may be either A or G.

**Table 1**

| | Sequence | SEQ ID NO: |
|---|---|---|
| Probes for CYP2C19*2 | 5'-cattgattatttcccAggaacccataac-3' | 13 |
| | 5'-attgattatttcccAggaacccataac-3' | 14 |
| | 5'-ttgattatttcccAggaacccataac-3' | 15 |
| | 5'-tgattatttcccAggaacccataac-3' | 16 |
| | 5'-gattatttcccAggaacccataac-3' | 17 |
| | 5'-attatttcccAggaacccataac-3' | 18 |
| | 5'-ttatttcccAggaacccataac-3' | 19 |
| | 5'-tatttcccAggaacccataac-3' | 20 |
| | 5'-atttcccAggaacccataac-3' | 21 |
| | 5'-tttcccAggaacccataac-3' | 22 |
| | 5'-ttcccAggaacccataac-3' | 23 |
| | 5'-tcccAggaacccataac-3' | 24 |
| | 5'-cccAggaacccataac-3' | 25 |

**Table 2**

| | Sequence | SEQ ID NO: |
|---|---|---|
| Probes for CYP2C19*3 | 5'-caccccctgAatccaggtaaggcca-3' | 26 |
| | 5'-caccccctgAatccaggtaaggcc-3' | 27 |
| | 5'-caccccctgAatccaggtaaggc-3' | 28 |
| | 5'-caccccctgAatccaggtaagg-3' | 29 |
| | 5'-caccccctgAatccaggtaag-3' | 30 |
| | 5'-caccccctgAatccaggtaa-3' | 31 |
| | 5'-caccccctgAatccaggta-3' | 32 |
| | 5'-caccccctgAatccaggt-3' | 33 |
| | 5'-ccctgAatccaggtaaggc-3' | 34 |
| | 5'-ccctgAatccaggtaagg-3' | 35 |
| | 5'-ccctgAatccaggtaag-3' | 36 |
| | 5'-ccctgAatccaggtaa-3' | 37 |
| | 5'-ccctgAatccaggta-3' | 38 |
| | 5'-ccctgAatccaggt-3' | 39 |

**Table 3**

| | Sequence | SEQ ID NO: |
|---|---|---|
| | 5'-cagagatActttgagaaca-3' | 40 |
| | 5'-cagagatActttaagaacag-3' | 41 |
| | 5'-cagagatActttgagaacaga-3' | 42 |
| | 5'-cagagatActttgagaacagaa-3' | 43 |
| | 5'-cagagatActttgagaacagaag-3' | 44 |
| | 5'-cagagatActttgagaacagaaga-3' | 45 |
| | 5'-cagagatActttgagaacagaagac-3' | 46 |
| | 5'-cagagatActtgagaacagaagaca-3' | 47 |
| | 5'-cagagatActttgagaacagaagacac-3' | 48 |
| | 5'-cagagatActttgagaacagaagacaca-3' | 49 |
| | 5'-cagagatActttgagaacaaagacacaa-3' | 50 |
| | 5'-tcagagatActttgagaac-3' | 51 |
| | 5'-atcagagatActttgagaac-3' | 52 |
| | 5'-catcagagatActttgagaac-3' | 53 |
| | 5'-acatcagagatActttgagaac-3' | 54 |
| Probes for CYP2C19*17 | 5'-tacatcagagatActttgagaac-3' | 55 |
| | 5'-ttacatcagagatActttgagaac-3' | 56 |
| | 5'-cttacatcagagatActttgagaac-3' | 57 |
| | 5'-tcttacatcagagatActttgagaac-3' | 58 |
| | 5'-ctcttacatcagagatActttgagaac-3' | 59 |
| | 5'-tctcttacatcagagatActttgagaac-3' | 60 |
| | 5'-atctcttacatcagagatActttgagaac-3' | 61 |
| | 5'-tatctcttacatcagagatActttgagaac-3' | 62 |
| | 5'-tcagagatActttgagaacagaagac-3' | 63 |
| | 5'-agapatActttgagaacagaagac-3' | 64 |
| | 5'-gagatActttgagaacagaagac-3' | 65 |
| | 5'-agatActttgagaacagaagac-3' | 66 |
| | 5'-gatActttgagaacagaagac-3' | 67 |
| | 5'-atActttgagaacagaagac-3' | 68 |
| | 5'-tActttgagaacagaagac-3' | 69 |

As described above, it is preferable that the probes for polymorphism analysis shown in Tables 1 to 3 are each labeled with a labeling substance such as a fluorescent dye.

Specific examples of the probe for CYP2C19*2, the probe for CYP2C19*3 and the probe for CYP2C19*17 also include complementary strands of the oligonucleotides shown in Tables 1 to 3.

It will be understood that the probes for polymorphism analysis to be used in the polymorphism analysis method of the present disclosure are not restricted to these specific exemplary embodiments.

### Drug Efficacy Evaluation Method

The drug efficacy evaluation method of the present disclosure includes at least: analyzing polymorphisms of the CYP2C19 gene by the polymorphism analysis method of the present disclosure; and evaluating the efficacy of a drug based on a result of the analysing. The method is applied to a sample from a patient to which the drug has been administered.

As described above, according to the polymorphism analysis method of the present disclosure, regions containing a site of gene polymorphism CYP2C19*2, CYP2C19*3 or CYP2C19*17, respectively, can be simultaneously and specifically amplified in a single reaction solution using a nucleic acid contained in a sample as a template and polymorphisms of the CYP2C19 gene can thus be analyzed. The gene polymorphisms CYP2C19*2, CYP2C19*3 and CYP2C19*17 are known to affect the in-blood dynamics and efficacies of various drugs such as proton pump inhibitors (e.g., omeprazole, esomeprazole, lansoprazole, rabeprazole and pantoprazole), diazepam, phenytoin, imipramine, hexobarbital, proguanil, carisoprodol, clopidogrel, voriconazole, nelfinavir, cyclophosphamide and tamoxifen. Thus, according to the drug efficacy evaluation method of the present disclosure, the efficacy of a drug can be evaluated based on the results of analyzing the polymorphisms of the CYP2C19 gene.

The thus obtained evaluation results can be used not only for estimation of the side effects of the drug but also for determination of therapeutic strategies including modification of dosage, shift to another drug and the like.

### Kit for Amplifying Cyp2c19 Gene

The kit of the present disclosure for amplifying target regions of the CYP2C19 gene for amplifying the CYP2C19*2 gene, which is hereinafter simply referred to as the kit of the present disclosure, includes at least the primer set of the present disclosure, and is used for amplifying target regions of the CYP2C19 gene. Since the kit of the present disclosure includes the primer set of the present disclosure, it is possible to specifically and efficiently amplify regions containing a site of gene polymorphism CYP2C19*2, CYP2C19*3 or CYP2C19*17, respectively.

In order to detect an amplification product obtained by a gene amplification method using the primer set of the present disclosure, it is preferable that the kit of the present disclosure further includes probes that are capable of hybridizing to a region containing a site of the gene polymorphism CYP2C19*2, CYP2C19*3 or CYP2C19*17, respectively (namely, the probe for CYP2C19*2, the probe for CYP2C19*3, and the probe for CYP2C19*17). As described above, it is preferable that these probes be fluorescently labeled probes. With regard to these probes, the matters described above in relation to the probes for polymorphism analysis can be applied as they are.

In the kit of the present disclosure, the primer sets (1) to (3) may be contained separately or in the form of a mixture. Further, the primer set of the present disclosure and the above-described probes may also be contained separately or in the form of a mixture.

The condition of being "contained separately" may be any condition in which the reagents are separated from each other such that they can be maintained in a non-contact state, and it is not necessarily required that the reagents be accommodated in separate containers that can be handled independently.

In addition to the above-described components, the kit of the present disclosure may also include various reagents that are required for the gene amplification method of the present disclosure or the polymorphism analysis method of the present disclosure. The kit of the present disclosure may further include, for example, an instruction manual which describes the gene amplification method of the present disclosure or the polymorphism analysis method of the present disclosure and an instruction manual which describes various reagents that are included or may be additionally included in the kit of the present disclosure. The invention also extends to use of the kit to amplify target regions in the CYP2C19 gene, to analyze polymorphisms in the CYP2C19 gene or to evaluate drug efficacy.

### EXAMPLES

The invention will now be described concretely by way of examples thereof; however, the invention is not restricted thereto.

In the following Examples, the primers shown in Table 4 were used as primers for amplifying target regions containing CYP2C19 gene polymorphism sites. All of these primers were prepared in accordance with an ordinary (conventional) method.

**Table 4**

| Name | Sequence (5'→3') | Number of bases | SEQ ID NO: |
|---|---|---|---|
| CYP2C19*2-F | taaattattgttttctcttagatatgcaataattttccca | 40 | 3 |
| CYP2C19*2-R | cccgagggttgttgatetccatc | 23 | 4 |
| CYP2C19*3-F | tgatggaaaaattgaatgaaaacatcaggattgta | 35 | 5 |
| CYP2C19*3-R | tcaaaaatgtacttcagggcttggtcaata | 30 | 6 |
| CYP2C19*17-F | gtttggaagttgttttgtttWgctaaaac (W = a/t) | 29 | 7 |
| CYP2C19*17-F7 | gcatctctggggctgttttcct | 22 | 8 |
| CYP2C19*17-F8 | tgtgaagcctgttttatgaacaggatgaat | 30 | 9 |
| CYP2C19*17-F9 | taatgtgaagcctgttttatgaacaggatg | 30 | 10 |
| CYP2C19*17-R | ctgggatttgagctgaggtcttctga | 26 | 11 |
| CYP2C19*17-R5 | tcttctgatgcccatcgtggc | 21 | 12 |

In the following Examples, the probes shown in Table 5 were used as the probe for CYP2C19*2, the probe for CYP2C19*3, or the probe for CYP2C19*17. All of these probes were prepared in accordance with an ordinary method. Cytosine (C) at the 3'- or 5'-end was labeled with BODIPY FL (registered trademark, manufactured by Molecular Probes, Inc.), TAMRA (registered trademark, manufactured by Molecular Probes, Inc.) or PACIFIC BLUE (registered trademark, manufactured by Molecular Probes, Inc.) in accordance with an ordinary method. For the probe 5T-CYP2C19*3, a phosphate group was added to the 3'-end in accordance with an ordinary method. In the base sequences shown in Table 5, the capitalized bases each indicate the site of the gene polymorphism CYP2C19*2, CYP2C19*3 or CYP2C19*17.

**Table 5**

| Name | Sequence (5'→3') | Number of bases | SEQ ID NO: |
|---|---|---|---|
| 3FL-CYP2C19*2 | tcccAggaacccataac-(BODIPY FL) | 17 | 24 |
| 5T-CYP2C19*3 | (TAMRA)-ccctgAatccaggtaag-P | 17 | 36 |
| 3PB-CYP2C19*17 | tcagagatActttgagaac-(PACIFIC BLUE) | 19 | 51 |

In the following Examples, an index called "amplification evaluation value" was used to evaluate the amplification efficiency of the primers. When this amplification evaluation value is about 2.5 or larger, it can be determined that an amplification product was obtained in a sufficient amount.

The amplification evaluation value is determined as follows.

First, PCR is performed using a PCR solution containing the respective primers and a fluorescence quenching probe. After the PCR, the reaction solution is heated and changes in fluorescence intensity associated with temperature increase are measured. Then, the amount of change in fluorescence intensity per unit time (primary differentiation value) is determined, and the secondary differentiation value is determined based on the primary differentiation value. As an example, it is assumed that such values of fluorescence intensity, primary differentiation and secondary differentiation as shown in Table 6 were obtained as a result of heating a post-PCR reaction solution from 40°C to 95°C. In Table 6, a value obtained by subtracting the fluorescence intensity at t₁ (°C) from the fluorescence intensity at t₁ + 1 (°C) (t₁ = 41°C to 94°C) is defined as the primary differentiation value at t₁ (°C), and a value obtained by subtracting the primary differentiation value at t₂ (°C) from the primary differentiation value at t₂ + 3 (°C) (t₂ = 41°C to 91°C) is defined as the secondary differentiation value at t₂ (°C).

**Table 6**

| Temperature (°C) | Fluorescence intensity | Primary Differentiation | Secondary Differentiation |
|---|---|---|---|
| 40 | 3088 | | |
| 41 | 3075 | -20 | -6 |
| 42 | 3055 | -24 | -8 |
| 43 | 3031 | -27 | -4 |
| 44 | 3004 | -26 | -8 |
| 45 | 2978 | -32 | 0 |
| 46 | 2946 | -31 | -1 |
| 47 | 2915 | -34 | 3 |
| 48 | 2881 | -32 | 2 |
| 49 | 2849 | -32 | 3 |
| 50 | 2817 | -31 | 2 |
| 51 | 2786 | -30 | 2 |
| 52 | 2756 | -29 | 0 |
| 53 | 2727 | -29 | 2 |
| 54 | 2698 | -28 | 3 |
| 55 | 2670 | -29 | 5 |
| 56 | 2641 | -27 | 4 |
| 57 | 2614 | -25 | 1 |
| 58 | 2589 | -24 | 2 |
| 59 | 2565 | -23 | 3 |
| 60 | 2542 | -24 | 5 |
| 61 | 2518 | -22 | 4 |
| 62 | 2496 | -20 | 4 |
| 63 | 2476 | -19 | 3 |
| 64 | 2457 | -18 | 5 |
| 65 | 2439 | -16 | 5 |
| 66 | 2423 | -16 | 7 |
| 67 | 2407 | -13 | 9 |
| 68 | 2394 | -11 | 10 |
| 69 | 2383 | -9 | 14 |
| 70 | 2374 | -4 | 17 |
| 71 | 2370 | -1 | 26 |
| 72 | 2369 | 5 | 50 |
| 73 | 2374 | 13 | 96 |
| 74 | 2387 | 25 | 106 |
| 75 | 2412 | 55 | 13 |
| 76 | 2467 | 109 | -106 |
| 77 | 2576 | 131 | -161 |
| 78 | 2707 | 68 | -106 |
| 79 | 2775 | 3 | -45 |
| 80 | 2778 | -30 | -9 |
| 81 | 2748 | -38 | -2 |
| 82 | 2710 | -42 | 5 |
| 83 | 2668 | -39 | 0 |
| 84 | 2629 | -40 | 2 |
| 85 | 2589 | -37 | -2 |
| 86 | 2552 | -39 | 3 |
| 87 | 2513 | -38 | 1 |
| 88 | 2475 | -39 | 8 |
| 89 | 2436 | -36 | 3 |
| 90 | 2400 | -37 | 6 |
| 91 | 2363 | -31 | 3 |
| 92 | 2332 | -33 | |
| 93 | 2299 | -31 | |
| 94 | 2268 | -28 | |
| 95 | 2240 | | |

Meanwhile, the minimum peak temperature Tₘᵢₙ, which is the Tm value representing the case where the fluorescence quenching probe in use and the sequence to be detected are a mismatch, and the maximum peak temperature Tₘₐₓ, which is the Tm value representing the case where the fluorescence quenching probe in use and the sequence to be detected are a perfect match, are determined in advance. It is assumed here that the minimum peak temperature Tₘᵢₙ is 59°C and the maximum peak temperature Tₘₐₓ is 77°C.

Next, with a range of from Tₘᵢₙ - 5 (°C) to Tₘₐₓ + 5 (°C) being defined as the search range, the maximum fluorescence intensity within this search range is determined. In the case of Table 6, the maximum fluorescence intensity in the search range of from 54°C to 82°C is 2,778.

Then, the maximum and minimum secondary differentiation values within a search range of from Tₘᵢₙ - 5 (°C) to Tₘₐₓ + 1 (°C) are determined. In the case of Table 6, the maximum and minimum secondary differentiation values in the search range of from 54°C to 78°C are 106 and -161, respectively.

Thereafter, the amplification evaluation value is calculated using the following equation:
Amplification evaluation value = 100 × (|Maximum secondary differentiation valuer| + |Minimum secondary differentiation value|)/Maximum fluorescence intensity
In the case of Table 6, the amplification evaluation value is: 100 × (106 + 161)/2778 ≈ 9.6.

### <Experimental Example 1>

E-Mix having the formulation shown in Table 7 and MK-Mix having the formulation shown in Table 8 were each prepared. Further, PreP-Mix having the formulation shown in Table 9 was prepared and, using this PreP-Mix, each P-Mix was prepared according to the formulations of Conditions 1 to 8 shown in Table 10. Then, the thus obtained E-Mix, P-Mix, MK-Mix and a human genomic DNA purified from whole blood were mixed as shown in Table 11 to prepare a reaction solution. In the human genomic DNA used here, all of CYP2C19*2, CYP2C19*3 and CYP2C19*17 were of wild-type (i.e., CYP2C19*2 was 681G/G, CYP2C19*3 was 636G/G, and CYP2C19*17 was -806C/C) (the same applies hereinafter).

**Table 7**

| E-Mix | For 1 test (µL) |
|---|---|
| Distilled water | 14.92 |
| 1M Tris-HCl (pH 8.6) | 0.49 |
| 20 w/v% BSA | 0.50 |
| 10 w/v% NaN₃ | 0.09 |
| 0.42 U/µL Taq polymerase | 2.00 |
| Total | 18.00 |

**Table 8**

| MK-Mix | For 1 test (µL) |
|---|---|
| Distilled water | 6.93 |
| 1M Tris-HCl (pH 8.6) | 0.27 |
| 1M KCl | 1.25 |
| 100 mM MgCl₂ | 1.50 |
| 10 w/v% NaN₃ | 0.05 |
| Total | 10.00 |

**Table 9**

| PreP-Mix | For 1 test (µL) |
|---|---|
| Distilled water | 2.92 |
| 80 w/v% glycerol | 2.50 |
| 1M Tris-HCl (pH 8.6) | 0.49 |
| 10 mM dNTP | 1.00 |
| 100 µM CYP2C19*2-F | 0.15 |
| 100 µM CYP2C19*2-R | 0.60 |
| 100 µM CYP2C19*3-F | 0.30 |
| 100 µM CYP2C19*3-R | 1.20 |
| 100 µM 3FL-CYP2C19*2 | 0.25 |
| 100 µM 5T-CYP2C19*3 | 0.25 |
| 100 µM 3PB-CYP2C19*17 | 0.25 |
| 10 w/v% NaN₃ | 0.09 |
| Total | 10.00 |

**Table 10**

| P-Mix | For 1 test (µL) | | | | | | | |
|---|---|---|---|---|---|---|---|---|
| | Condition 1 | Condition 2 | Condition 3 | Condition 4 | Condition 5 | Condition 6 | Condition 7 | Condition 8 |
| PreP-Mix | 10.00 | 10.00 | 10.00 | 10.00 | 10.00 | 10.00 | 10.00 | 10.00 |
| Distilled water | 6.00 | 6.00 | 5.95 | 5.95 | 6.00 | 6.00 | 6.00 | 6.00 |
| 100 µM CYP2C19*17-F | 1.60 | 1.60 | | | | | | |
| 100 µM CYP2C19*17-F7 | | | 1.65 | 1.65 | | | | |
| 100 µM CYP2C19*17-F8 | | | | | 1.60 | 1.60 | | |
| 100 µM CYP2C19*17-F9 | | | | | | | 1.60 | 1.60 |
| 100 µM CYP2C19*17-R | 0.40 | | 0.40 | | 0.40 | | 0.40 | |
| 100 µM CYP2C19*17-R5 | | 0.40 | | 0.40 | | 0.40 | | 0.40 |
| Total | 18.00 | 18.00 | 18.00 | 18.00 | 18.00 | 18.00 | 18.00 | 18.00 |

**Table 11**

| Reaction solution | For 1 test (µL) |
|---|---|
| E-Mix | 18.00 |
| P-Mix (Conditions 1 to 8) | 18.00 |
| MK-Mix | 10.00 |
| 25 copies/µL human genomic DNA | 4.00 |
| Total | 50.00 |

The thus prepared reaction solution was subjected to PCR and Tm analysis using a fully-automated SNPs analyzer (trade name: I-DENSY IS-5320, manufactured by ARKRAY, Inc.). Measurement was performed once at each reaction temperature for each reaction solution (n = 1).

The PCR was performed by treating the reaction solution at 95°C for 60 seconds and subsequently repeating 50 cycles of a 1-second treatment at 95°C and a 30-second treatment at t°C. The reaction temperature, t, was set at 4 levels of 58°C, 60°C, 62°C and 64°C.

The Tm analysis was performed by treating the reaction solution at 95°C for 1 second and then at 40°C for 60 seconds and subsequently measuring changes in fluorescence intensity with time during a period in which the temperature of the reaction solution was raised from 40°C to 95°C at a rate of 1°C/3 seconds. In the Tm analysis, the fluorescent dye BODIPY FL had an excitation wavelength of 420 nm to 485 nm and a detection wavelength of 520 to 555 nm; the fluorescent dye TAMRA had an excitation wavelength of 520 nm to 555 nm and a detection wavelength of 585 nm to 700 nm; and the fluorescent dye PACIFIC BLUE had an excitation wavelength of 365 nm to 415 nm and a detection wavelength of 445 nm to 480 nm.

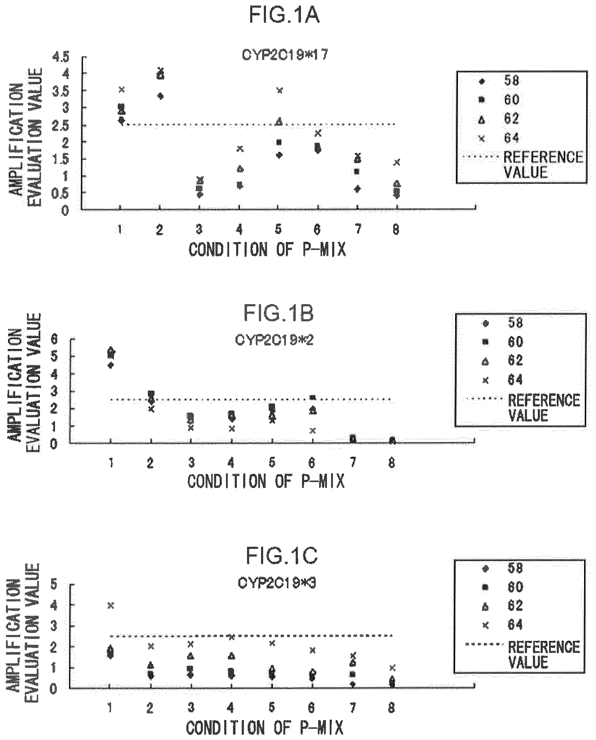

FIG. 1A shows the amplification evaluation values of a target region containing a site of gene polymorphism CYP2C19*17. FIG. 1B shows the amplification evaluation values of a target region containing a site of gene polymorphism CYP2C19*2. FIG. 1C shows the amplification evaluation values of a target region containing a site of gene polymorphism CYP2C19*3.

As shown in FIG. 1A, for CYP2C19*17, the amplification evaluation value was 2.5 or larger when the P-Mix was in the Conditions 1,2 and 5. As shown in FIG. 1B, for CYP2C19*2, the amplification evaluation value was 2.5 or larger when the P-Mix was in the Conditions 1 and 2. As shown in FIG. 1C, for CYP2C19*3, the amplification evaluation value was 2.5 or larger when the P-Mix was in the Condition 1. From the results shown in FIGs. 1A to 1C, it is believed that an amplification evaluation value of 2.5 or larger can be obtained for all of CYP2C19*17, CYP2C19*2 and CYP2C19*3 by increasing the amount of DNA polymerase in the Conditions 1,2 and 5 of the P-Mix. Among the Conditions 1,2 and 5 of the P-Mix, particularly in the Condition 2, the effect of changing the reaction temperature was small and the tolerability against the changes in reaction temperature was high.

### <Experimental Example 2>

PreE-Mix having the formulation shown in Table 12 was prepared and, using this PreE-Mix, each E-Mix was prepared according to the formulations of Conditions 1 and 2 shown in Table 13. In addition, PreP-Mix having the formulation shown in Table 9 was prepared and, using this PreP-Mix, each P-Mix was prepared according to the formulations of Conditions 1,2 and 5 shown in Table 10. Further, MK-Mix having the formulation shown in Table 8 was prepared. Then, the thus obtained E-Mix, P-Mix, MK-Mix and a human genomic DNA purified from whole blood were mixed as shown in Table 14 to prepare a reaction solution.

**Table 12**

| PreE-Mix | For 1 test (µL) |
|---|---|
| Distilled water | 8.92 |
| 1M Tris-HCl (pH 8.6) | 0.49 |
| 20 w/v% BSA | 0.50 |
| 10 w/v% NaN₃ | 0.09 |
| Total | 10.00 |

**Table 13**

| E-Mix | For 1 test (µL) | |
|---|---|---|
| | Condition 1 | Condition 2 |
| PreE-Mix | 10.00 | 10.00 |
| Distilled water | 6.00 | 4.00 |
| 0.42 U/µL Taq polymerase | 2.00 | 4.00 |
| Total | 18.00 | 18.00 |

**Table 14**

| Reaction solution | For 1 test (µL) |
|---|---|
| E-Mix (Condition 1 or 2) | 18.00 |
| P-Mix (Condition 1, 2 or 5) | 18.00 |
| MK-Mix | 10.00 |
| 25 copies/µL human genomic DNA | 4.00 |
| Total | 50.00 |

The thus prepared reaction solution was subjected to PCR and Tm analysis using a fully-automated SNPs analyzer (trade name: I-DENSY IS-5320, manufactured by ARKRAY, Inc.). Measurement was performed four times for each reaction solution (n = 4).

The PCR was performed by treating the reaction solution at 95°C for 60 seconds and subsequently repeating 50 cycles of a 1-second treatment at 95°C and a 30-second treatment at 64°C.

The Tm analysis was performed by treating the reaction solution at 95°C for 1 second and then at 40°C for 60 seconds and subsequently measuring changes in fluorescence intensity with time during a period in which the temperature of the reaction solution was raised from 40°C to 95°C at a rate of 1°C/3 seconds.

The reaction conditions (combinations of E-Mix and P-Mix) in Experimental Example 2 were as shown in Table 15.

**Table 15**

| Reaction condition | | | | | |
|---|---|---|---|---|---|
| 1 | 2 | 3 | 4 | 5 | 6 |
| E-Mix Condition 1 | | | E-Mix Condition 2 | | |
| P-Mix Condition 1 | P-Mix Condition 2 | P-Mix Condition 5 | P-Mix Condition 1 | P-Mix Condition 2 | P-Mix Condition 5 |

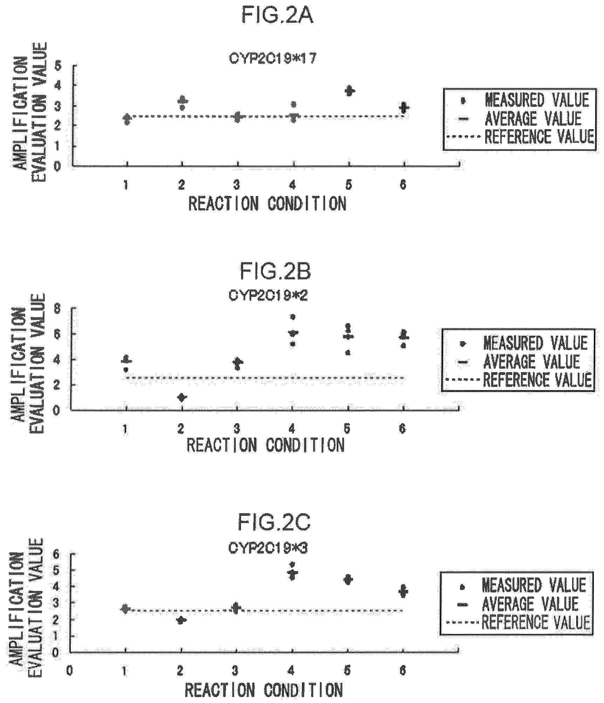

FIG. 2A shows the amplification evaluation values of a target region containing a site of gene polymorphism CYP2C19*17. FIG. 2B shows the amplification evaluation values of a target region containing a site of gene polymorphism CYP2C19*2. FIG. 2C shows the amplification evaluation values of a target region containing a site of gene polymorphism CYP2C19*3.

As shown in FIGs. 2A to 2C, in the reaction conditions 4 to 6 (Condition 2 of E-Mix) where the amount of DNA polymerase was doubled, the average amplification evaluation value was 2.5 or larger for all of CYP2C19*17, CYP2C19*2 and CYP2C19*3. Particularly, in the reaction conditions 5 and 6 (Conditions 2 and 5 of P-Mix), an amplification evaluation value of 2.5 or larger was obtained in all of the four measurements. Comparing the reaction conditions 5 and 6, the reaction condition 5 is believed to be more preferable based on the amplification evaluation value.

### <Experimental Example 3>

E-Mix having the formulation shown in Table 16 and P-Mix having the formulation shown in Table 17 were each prepared. In addition, MK-Mix having the formulation shown in Table 8 was prepared. Then, the thus obtained E-Mix, P-Mix, MK-Mix and a human genomic DNA purified from whole blood were mixed as shown in Table 18 to prepare a reaction solution.

**Table 16**

| E-Mix | For 1 test (µL) |
|---|---|
| Distilled water | 14.73 |
| 1M Tris-HCl (pH 8.6) | 0.49 |
| 20 w/v% BSA | 0.50 |
| 10 w/v% NaN₃ | 0.09 |
| 0.86 U/µL Taq polymerase | 2.19 |
| Total | 18.00 |

**Table 17**

| P-Mix | For 1 test (µL) |
|---|---|
| Distilled water | 8.92 |
| 80 w/v% glycerol | 2.50 |
| 1M Tris-HCl (pH 8.6) | 0.49 |
| 10 mM dNTP | 1.00 |
| 100 µM CYP2C19*2-F | 0.15 |
| 100 µM CYP2C19*2-R | 0.60 |
| 100 µM CYP2C19*3-F | 0.30 |
| 100 µM CYP2C19*3-R | 1.20 |
| 100 µM CYP2C19*17-F | 1.60 |
| 100 µM CYP2C19*17-R5 | 0.40 |
| 100 µM 3FL-CYP2C19*2 | 0.25 |
| 100 µM 5T-CYP2C19*3 | 0.25 |
| 100 µM 3PB-CYP2C19*17 | 0.25 |
| 10 w/v% NaN₃ | 0.09 |
| Total | 18.00 |

**Table 18**

| Reaction solution | For 1 test (µL) |
|---|---|
| E-Mix | 18.00 |
| P-Mix | 18.00 |
| MK-Mix | 10.00 |
| 25 copies/µL human genomic DNA | 4.00 |
| Total | 50.00 |

The thus prepared reaction solution was subjected to PCR and Tm analysis using a fully-automated SNPs analyzer (trade name: I-DENSY IS-5320, manufactured by ARKRAY, Inc.). Measurement was performed four times at each temperature for each reaction solution (n = 4).

The PCR was performed by treating the reaction solution at 95°C for 60 seconds and subsequently repeating 50 cycles of a 1-second treatment at 95°C and a 30-second treatment at t°C. The reaction temperature, t, was set at 5 levels of 58°C, 60°C, 62°C, 64°C and 66°C.

The Tm analysis was performed by treating the reaction solution at 95°C for 1 second and then at 40°C for 60 seconds and subsequently measuring changes in fluorescence intensity with time during a period in which the temperature of the reaction solution was raised from 40°C to 95°C at a rate of 1°C/3 seconds.

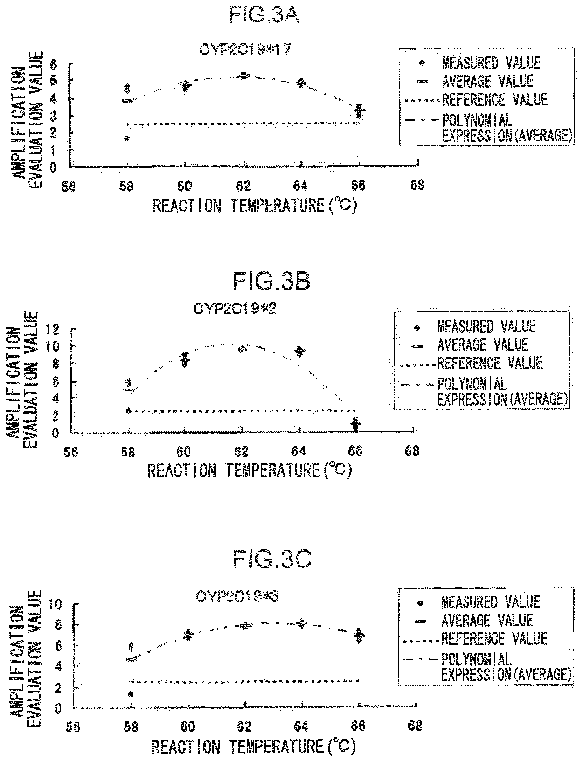

FIG. 3A shows the amplification evaluation values of a target region containing a site of gene polymorphism CYP2C19*17. FIG. 3B shows the amplification evaluation values of a target region containing a site of gene polymorphism CYP2C19*2. FIG. 3C shows the amplification evaluation values of a target region containing a site of gene polymorphism CYP2C19*3.

As shown in FIGs. 3A to 3C, particularly at a reaction temperature of 62°C, the amplification evaluation values were stable for all of CYP2C19*17, CYP2C19*2 and CYP2C19*3 and tolerance was exhibited for reaction temperatures of at least ±2°C.

### <Experimental Example 4>

E-Mix having the formulation shown in Table 16, P-Mix having the formulation shown in Table 17 and MK-Mix having the formulation shown in Table 8 were each prepared.

Meanwhile, a sample of whole blood 1 containing heterozygote-type (Ht) CYP2C19*17 and CYP2C19*2 (that is, CYP2C19*17 was -806C/T and CYP2C19*2 was 681G/A) and a wild-type (WT) CYP2C19*3 (that is, 636G/G) and a sample of whole blood 2 containing a wild-type (WT) CYP2C19*17 (that is, -806C/C) and heterozygote-type (Ht) CYP2C19*2 and CYP2C19*3 (that is, CYP2C19*2 was 681G/A and CYP2C19*3 was 636G/A) were prepared. Then, 20 µL of each of the whole blood samples 1 or 2 were respectively mixed with 140 µL of the below-described diluent A, and 20 µL of each of the resulting mixtures were further respectively mixed with 140 µL of the below-described diluent B. By heating 17 µL of the thus obtained mixtures at 95°C for 10 minutes, 4 µL of pre-treated whole blood samples were obtained respectively.
(Diluent A)
   10 mM Tris-HCl (pH 8.0), 0.1 mM EDTA, 0.05% NaN₃ and 0.3% SDS
(Diluent B)
   10 mM Tris-HCl (pH 8.0), 0.1 mM EDTA and 0.05% NaN₃

Thereafter, the thus obtained E-Mix, P-Mix, MK-Mix and pre-treated whole blood 1 or 2 were mixed as shown in Table 19 to prepare a reaction solution.

**Table 19**

| Reaction solution | For 1 test (µL) |
|---|---|
| E-Mix | 18.00 |
| P-Mix | 18.00 |
| MK-Mix | 10.00 |
| Pre-treated whole blood samples 1 or 2 | 4.00 |
| Total | 50.00 |

The thus prepared reaction solution was subjected to PCR and Tm analysis using a fully-automated SNPs analyzer (trade name: I-DENSY IS-5320, manufactured by ARKRAY, Inc.).

The PCR was performed by treating the reaction solution at 95°C for 60 seconds and subsequently repeating 50 cycles of a 1-second treatment at 95°C and a 30-second treatment at 62°C.

The Tm analysis was performed by treating the reaction solution at 95°C for 1 second and then at 40°C for 60 seconds and subsequently measuring changes in fluorescence intensity with time during a period in which the temperature of the reaction solution was raised from 40°C to 95°C at a rate of 1°C/3 seconds.

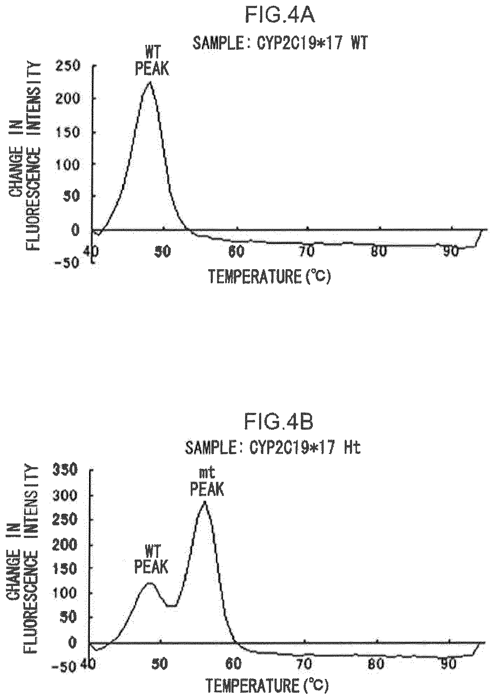

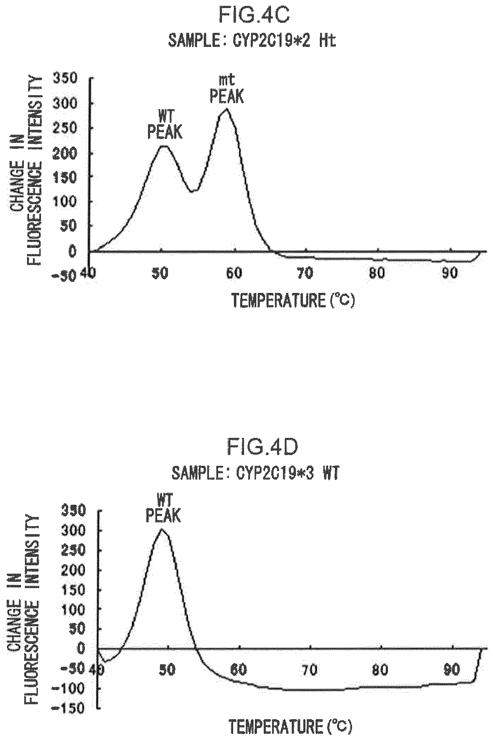

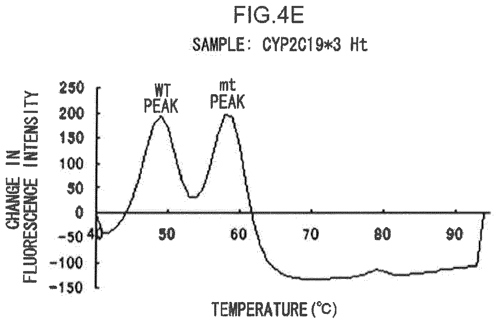

FIGs. 4A and 4B each show the results of Tm analysis where the fluorescence intensity was measured at a detection wavelength corresponding to the probe 3PB-CYP2C19*17 for the reaction solutions prepared by using the whole blood 2 or 1. FIG. 4C shows the results of Tm analysis where the fluorescence intensity was measured at a detection wavelength corresponding to the probe 3FL-CYP2C19*2 for the reaction solution prepared by using the whole blood 2. FIGs. 4D and 4E each show the results of Tm analysis where the fluorescence intensity was measured at a detection wavelength corresponding to the probe 5T-CYP2C19*3 for the reaction solutions prepared by using the whole blood 1 or 2.

As shown in FIGs. 4A to 4E, only one peak was observed for the wild-type (WT) samples, while two peaks of a wild type (WT) and a mutant type (mt) were observed for the heterozygote-type (Ht) samples. Therefore, the genotypes can be determined based on these results of Tm analysis.

## Claims

1. A kit for amplifying target regions of the CYP2C19 gene by a nucleic acid amplification method, the kit comprising (i) a primer set comprising a primer set (1), a primer set (2), and a primer set (3) that is selected from the group consisting of primer sets (3a), (3b) and (3c),
the primer set (1) comprising a forward primer that is an oligonucleotide consisting of a base sequence represented by SEQ ID NO:3, and a reverse primer that is an oligonucleotide consisting of a base sequence represented by SEQ ID NO:4,
the primer set (2) comprising a forward primer that is an oligonucleotide consisting of a base sequence represented by SEQ ID NO:5, and a reverse primer that is an oligonucleotide consisting of a base sequence represented by SEQ ID NO:6,
the primer set (3a) comprising a forward primer that is an oligonucleotide consisting of a base sequence represented by SEQ ID NO:7, and a reverse primer that is an oligonucleotide consisting of a base sequence represented by SEQ ID NO:11,
the primer set (3b) comprising a forward primer that is an oligonucleotide consisting of a base sequence represented by SEQ ID NO:7, and a reverse primer that is an oligonucleotide consisting of a base sequence represented by SEQ ID NO:12, and
the primer set (3c) comprising a forward primer that is an oligonucleotide consisting of a base sequence represented by SEQ ID NO:9, and a reverse primer that is an oligonucleotide consisting of a base sequence represented by SEQ ID NO:11; and (ii) a probe that is an oligonucleotide consisting of a base sequence represented by SEQ ID NO: 24, a probe that is an oligonucleotide consisting of a base sequence represented by SEQ ID NO: 36, and a probe that is an oligonucleotide consisting of a base sequence represented by SEQ ID NO: 51.

2. The kit according to claim 1, wherein the primer set comprises the primer sets (1), (2) and (3b).

3. The kit according to claim 1 or 2, wherein the probes are fluorescently labelled probes.

4. A polymorphism analysis method comprising:
amplifying target regions of the CYP2C19 gene by a gene amplification method comprising amplifying target regions in the CYP2C19 gene in a single reaction solution by using a nucleic acid contained in a sample as a template and the primer set of the kit according to any one of claims 1 to 3;
forming hybrids between single-stranded amplified nucleic acids that are amplification products obtained by the amplification and the probes of the kit of any one of claims 1 to 3;
changing the temperature of a reaction solution containing the hybrids and measuring changes in signals reflecting dissociation of the hybrids; and
analyzing polymorphisms of the CYP2C19 gene based on the changes in the signals.

5. The method according to claim 4, wherein the sample is a biological sample.

6. The method according to claim 5, wherein the biological sample is whole blood.

7. The method according to any one of claims 4 to 6, wherein the ratio of the total molar amount of the primer set (1) to the total molar amount of the primer set (2) (primer set (1): primer set (2)) is from 1:1 to 1:3, and the ratio of the total molar amount of the primer set (1) to the total molar amount of the primer set (3) (primer set (1): primer set (3)) is from 1:1.5 to 1:4.

8. The method according to any one of claims 4 to 7, wherein the amplifying of the target regions and the forming of hybrids proceed simultaneously.

9. Use of a kit to amplify target regions in the CYP2C19 gene, to analyze polymorphisms in the CYP2C19 gene or to evaluate drug efficacy, wherein said kit is as defined in any one of claims 1 to 3.

## Patentansprüche

1. Kit zum Amplifizieren von Zielregionen des CYP2C19-Gens durch ein Nukleinsäure-Amplifikationsverfahren, wobei das Kit
(i) einen Primersatz, der einen Primersatz (1), einen Primersatz (2) und einen Primersatz (3) umfasst, der ausgewählt ist aus der Gruppe bestehend aus den Primersätzen (3a), (3b) und (3c),
wobei der Primersatz (1) einen Vorwärtsprimer, der ein Oligonukleotid ist, bestehend aus einer Basensequenz, dargestellt durch SEQ ID Nr.:3, und einen Rückwärtsprimer, der ein Oligonukleotid ist, bestehend aus einer Basensequenz, dargestellt durch SEQ ID Nr.:4, umfasst,
wobei der Primersatz (2) einen Vorwärtsprimer, der ein Oligonukleotid ist, bestehend aus einer Basensequenz, dargestellt durch SEQ ID Nr.:5, und einen Rückwärtsprimer, der ein Oligonukleotid ist, bestehend aus einer Basensequenz, dargestellt durch SEQ ID Nr.:6, umfasst,
wobei der Primersatz (3a) einen Vorwärtsprimer, der ein Oligonukleotid ist, bestehend aus einer Basensequenz, dargestellt durch SEQ ID Nr.:7, und einen Rückwärtsprimer, der ein Oligonukleotid ist, bestehend aus einer Basensequenz, dargestellt durch SEQ ID Nr.:11, umfasst,
wobei der Primersatz (3b) einen Vorwärtsprimer, der ein Oligonukleotid ist, bestehend aus einer Basensequenz, dargestellt durch SEQ ID Nr.:7, und einen Rückwärtsprimer, der ein Oligonukleotid ist, bestehend aus einer Basensequenz, dargestellt durch SEQ ID Nr.:12, umfasst, und
wobei der Primersatz (3c) einen Vorwärtsprimer, der ein Oligonukleotid ist, bestehend aus einer Basensequenz, dargestellt durch SEQ ID Nr.:9, und einen Rückwärtsprimer, der ein Oligonukleotid ist, bestehend aus einer Basensequenz, dargestellt durch SEQ ID Nr.:11, umfasst; und
(ii) eine Sonde, die ein Oligonukleotid ist, bestehend aus einer Basensequenz, dargestellt durch SEQ ID Nr.: 24, eine Sonde, die ein Oligonukleotid ist, bestehend aus einer Basensequenz, dargestellt durch SEQ ID Nr.: 36, und eine Sonde, die ein Oligonukleotid ist, bestehend aus einer Basensequenz, dargestellt durch SEQ ID Nr.: 51, umfasst.

2. Kit nach Anspruch 1, wobei der Primersatz die Primersätze (1), (2) und (3b) umfasst.

3. Kit nach Anspruch 1 oder 2, wobei die Sonden fluoreszenzmarkierte Sonden sind.

4. Polymorphismusanalyseverfahren, umfassend:
Amplifizieren von Zielregionen des CYP2C19-Gens durch ein Genamplifikationsverfahren, umfassend das Amplifizieren von Zielregionen im CYP2C19-Gen in einer einzigen Reaktionslösung unter Verwendung einer in einer Probe enthaltenen Nukleinsäure als eine Matrize und des Primersatzes des Kits nach einem der Ansprüche 1 bis 3;
Bilden von Hybriden zwischen einzelsträngigen amplifizierten Nukleinsäuren, die durch die Amplifikation erhaltene Amplifikationsprodukte sind, und den Sonden des Kits nach einem der Ansprüche 1 bis 3;
Ändern der Temperatur einer Reaktionslösung, die die Hybride enthält und Messen von Signaländerungen, die die Dissoziation der Hybride widerspiegeln; und
Analysieren von Polymorphismen des CYP2C19-Gens, basierend auf den Signaländerungen.

5. Verfahren nach Anspruch 4, wobei die Probe eine biologische Probe ist.

6. Verfahren nach Anspruch 5, wobei die biologische Probe Vollblut ist.

7. Verfahren nach einem der Ansprüche 4 bis 6, wobei das Verhältnis der Gesamtmolmenge des Primersatzes (1) zur Gesamtmolmenge des Primersatzes (2) (Primersatz (1):Primersatz (2)) von 1:1 bis 1:3 reicht, und das Verhältnis der Gesamtmolmenge des Primersatzes (1) zur Gesamtmolmenge des Primersatzes (3) (Primersatz (1):Primersatz (3)) von 1:1,5 bis 1:4 reicht.

8. Verfahren nach einem der Ansprüche 4 bis 7, wobei das Amplifizieren der Zielregionen und das Bilden von Hybriden gleichzeitig ablaufen.

9. Verwendung eines Kits, um Zielregionen im CYP2C19-Gen zu amplifizieren, um Polymorphismen im CYP2C19-Gen zu analysieren oder um eine Arzneimittelwirksamkeit zu bewerten, wobei das Kit beschaffen ist, wie in einem der Ansprüche 1 bis 3 definiert.

## Revendications

1. Kit pour amplifier des régions cibles du gène CYP2C19 par un procédé d'amplification d'acide nucléique, le kit comprenant
(i) un jeu d'amorces comprenant un jeu d'amorces (1), un jeu d'amorces (2), et un jeu d'amorces (3) qui est sélectionné dans le groupe constitué des jeux d'amorces (3a), (3b) et (3c),
le jeu d'amorces (1) comprenant une amorce sens qui est un oligonucléotide consistant en une séquence de bases représentée par SEQ ID NO : 3, et une amorce antisens qui est un oligonucléotide consistant en une séquence de bases représentée par SEQ ID NO : 4,
le jeu d'amorces (2) comprenant une amorce sens qui est un oligonucléotide consistant en une séquence de bases représentée par SEQ ID NO : 5, et une amorce antisens qui est un oligonucléotide consistant en une séquence de bases représentée par SEQ ID NO : 6,
le jeu d'amorces (3a) comprenant une amorce sens qui est un oligonucléotide consistant en une séquence de bases représentée par SEQ ID NO : 7, et une amorce antisens qui est un oligonucléotide consistant en une séquence de bases représentée par SEQ ID NO : 11,
le jeu d'amorces (3b) comprenant une amorce sens qui est un oligonucléotide consistant en une séquence de bases représentée par SEQ ID NO : 7, et une amorce antisens qui est un oligonucléotide consistant en une séquence de bases représentée par SEQ ID NO : 12, et
le jeu d'amorces (3c) comprenant une amorce sens qui est un oligonucléotide consistant en une séquence de bases représentée par SEQ ID NO : 9, et une amorce antisens qui est un oligonucléotide consistant en une séquence de bases représentée par SEQ ID NO : 11 ; et
(ii) une sonde qui est un oligonucléotide consistant en une séquence de bases représentée par SEQ ID NO : 24, une sonde qui est un oligonucléotide consistant en une séquence de bases représentée par SEQ ID NO : 36, et une sonde qui est un oligonucléotide consistant en une séquence de bases représentée par SEQ ID NO : 51.

2. Kit selon la revendication 1, dans lequel le jeu d'amorces comprend les jeux d'amorces (1), (2) et (3b).

3. Kit selon la revendication 1 ou 2, dans lequel les sondes sont des sondes à marquage fluorescent.

4. Procédé d'analyse de polymorphismes comprenant :
l'amplification de régions cibles du gène CYP2C19 par un procédé d'amplification de gène comprenant l'amplification de régions cibles du gène CYP2C19 dans une seule solution réactionnelle en utilisant un acide nucléique contenu dans un échantillon comme matrice et le jeu d'amorces du kit selon l'une quelconque des revendications 1 à 3;
la formation d'hybrides entre des acides nucléiques amplifiés à un seul brin qui sont des produits d'amplification obtenus par l'amplification et les sondes du kit selon l'une quelconque des revendications 1 à 3 ;
le changement de la température d'une solution réactionnelle contenant les hybrides et la mesure des changements de signaux reflétant la dissociation des hybrides ; et
l'analyse des polymorphismes du gène CYP2C19 sur la base des changements des signaux.

5. Procédé selon la revendication 4, dans lequel l'échantillon est un échantillon biologique.

6. Procédé selon la revendication 5, dans lequel l'échantillon biologique est du sang entier.

7. Procédé selon l'une quelconque des revendications 4 à 6, dans lequel le rapport entre la quantité molaire totale du jeu d'amorces (1) et la quantité molaire totale du jeu d'amorces (2) (jeu d'amorces (1) : jeu d'amorces (2)) est de 1 : 1 à 1 : 3, et le rapport entre la quantité molaire totale du jeu d'amorces (1) et la quantité molaire totale du jeu d'amorces (3) (jeu d'amorces (1) : jeu d'amorces (3)) est de 1 : 1,5 à 1 : 4.

8. Procédé selon l'une quelconque des revendications 4 à 7, dans lequel l'amplification des régions cibles et la formation des hybrides s'effectuent simultanément.

9. Utilisation d'un kit pour amplifier des régions cibles dans le gène CYP2C19, pour analyser des polymorphismes dans le gène CYP2C19 ou pour évaluer l'efficacité d'un médicament, dans laquelle ledit kit est tel que défini dans l'une quelconque des revendications 1 à 3.
